(19) Europäisches Patentamt
European Patent Office
Office européen des brevets

(11) EP 3 144 000 A1

(12) EUROPEAN PATENT APPLICATION

(43) Date of publication:
22.03.2017 Bulletin 2017/12

(21) Application number: 16187550.5

(22) Date of filing: 11.02.2010

(51) Int Cl.:
A61K 31/7048 (2006.01) A61K 31/522 (2006.01)
A61P 3/06 (2006.01) A61P 3/08 (2006.01)
A61P 3/10 (2006.01) A61K 9/20 (2006.01)

(84) Designated Contracting States:
AT BE BG CH CY CZ DE DK EE ES FI FR GB GR HR HU IE IS IT LI LT LU LV MC MK MT NL NO PL PT RO SE SI SK SM TR
Designated Extension States:
BA RS

(30) Priority: 13.02.2009 US 152306 P

(62) Document number(s) of the earlier application(s) in accordance with Art. 76 EPC:
10703474.6 / 2 395 984

(27) Previously filed application:
11.02.2010 PCT/EP2010/051735

(71) Applicant: Boehringer Ingelheim International GmbH
55216 Ingelheim am Rhein (DE)

(72) Inventor: EISENREICH, Wolfram
55216 INGELHEIM AM RHEIN (DE)

(74) Representative: Simon, Elke Anna Maria et al
Boehringer Ingelheim GmbH
Binger Strasse 173
55216 Ingelheim am Rhein (DE)

Remarks:
This application was filed on 07-09-2016 as a divisional application to the application mentioned under INID code 62.

# (54) PHARMACEUTICAL COMPOSITION COMPRISING LINAGLIPTIN AND A SGLT2 INHIBITOR, AND USES THEREOF

(57) The present invention relates to pharmaceutical compositions of linagliptin, pharmaceutical dosage forms, their preparation, their use and methods for treating metabolic disorders.

EP 3 144 000 A1

## Description

### Technical Field of the Invention

The present invention relates to pharmaceutical compositions comprising linagliptin as a first active pharmaceutical ingredients. Furthermore the present invention relates to a pharmaceutical dosage form comprising such a pharmaceutical composition. In addition the invention relates to a process for the preparation of such a pharmaceutical dosage form. In addition the invention relates to the use of the pharmaceutical composition and of the pharmaceutical dosage form in the treatment and/or prevention of selected diseases and medical conditions, in particular of one or more conditions selected from type 1 diabetes mellitus, type 2 diabetes mellitus, impaired glucose tolerance, impaired fasting blood glucose and hyperglycemia inter alia. Furthermore the present invention relates to methods of treating and/or preventing of such diseases and medical conditions wherein a pharmaceutical composition or pharmaceutical dosage form according to the invention is administered to a patient in need thereof.

### Background of the Invention

The compound linagliptin is a DPP-IV inhibitor. The enzyme DPP-IV (dipeptidyl peptidase IV) also known as CD26 is a serine protease known to lead to the cleavage of a dipeptide from the N-terminal end of a number of proteins having at their N-terminal end a prolin or alanin residue. Due to this property DPP-IV inhibitors interfere with the plasma level of bioactive peptides including the peptide GLP-1 and are considered to be promising drugs for the treatment of diabetes mellitus, in particular type 2 diabetes mellitus.

In attempts to prepare pharmaceutical compositions of selected DPP-IV inhibitors, such as linagliptin, it has been observed, that the DPP-IV inhibitors with a primary or secondary amino group show incompatibilities, degradation problems, or extraction problems with a number of customary excipients such as microcrystalline cellulose, sodium starch glycolate, croscarmellose sodium, tartaric acid, citric acid, glucose, fructose, saccharose, lactose, maltodextrines. Though the compounds themselves are very stable, they react with many excipients used in solid dosage forms and with impurities of excipients, especially in tight contact provided in tablets and at high excipient/drug ratios. The amino group appears to react with reducing sugars and with other reactive carbonyl groups and with carboxylic acid functional groups formed for example at the surface of microcrystalline cellulose by oxidation. These unforeseen difficulties are primarily observed in low dosage ranges which are required due to the surprising potency of the selected inhibitors, such as linagliptin. Thus, pharmaceutical compositions are required so solve these technical problems associated with the unexpected potency of selected DPP-IV inhibitor compounds. Pharmaceutical compositions comprising linagliptin as the only active pharmaceutical ingredient are described in the WO 2007/128724.

Type 2 diabetes is an increasingly prevalent disease that due to a high frequency of complications leads to a significant reduction of life expectancy. Because of diabetes-associated microvascular complications, type 2 diabetes is currently the most frequent cause of adult-onset loss of vision, renal failure, and amputations in the industrialized world. In addition, the presence of type 2 diabetes is associated with a two to five fold increase in cardiovascular disease risk.

After long duration of disease, most patients with type 2 diabetes will eventually fail on oral therapy and become insulin dependent with the necessity for daily injections and multiple daily glucose measurements.

Oral antidiabetic drugs conventionally used in therapy (such as e.g. first- or second-line, and/or mono- or (initial or add-on) combination therapy) include, without being restricted thereto, metformin, sulphonylureas, thiazolidinediones, glinides and $\alpha$-glucosidase inhibitors.

The high incidence of therapeutic failure is a major contributor to the high rate of long-term hyperglycemia-associated complications or chronic damages (including micro- and macrovascular complications such as e.g. diabetic nephrophathy, retinopathy or neuropathy, or cardiovascular complications) in patients with type 2 diabetes.

Therefore, there is an unmet medical need for methods, medicaments and pharmaceutical compositions with a good efficacy with regard to glycemic control, with regard to disease-modifying properties and with regard to reduction of cardiovascular morbidity and mortality while at the same time showing an improved safety profile.

SGLT2 inhibitors inhibitors represent a novel class of agents that are being developed for the treatment or improvement in glycemic control in patients with type 2 diabetes. Glucopyranosyl-substituted benzene derivative are described in the prior art as SGLT2 inhibitors, for example in WO 01/27128, WO 03/099836, WO 2005/092877, WO 2006/034489, WO 2006/064033, WO 2006/117359, WO 2006/117360, WO 2007/025943, WO 2007/028814, WO 2007/031548, WO 2007/093610, WO 2007/128749, WO 2008/049923, WO 2008/055870, WO 2008/055940. The glucopyranosyl-substituted benzene derivatives are proposed as inducers of urinary sugar excretion and as medicaments in the treatment of diabetes.

**Aim of the present invention**

The aim of the present invention is to provide a pharmaceutical composition comprising linagliptin which shows no signs or only marginal signs of degradation of linagliptin and thus enables a good to very good shelf life.

Another aim of the invention is to provide a pharmaceutical composition comprising linagliptin which has high content uniformity and/or which allows an effective production with regard to time and costs of pharmaceutical dosage forms.

Another aim of the invention is to provide a pharmaceutical dosage form comprising linagliptin which has a good shelf life, which has a short disintegration time, which has good dissolution properties and/or which enables a high bioavailability of linaglitpin in a patient.

A further aim of the present invention is to provide a pharmaceutical composition comprising a combination of a DPPIV inhibitor and an SGLT2 inhibitor.

Another aim of the present invention is to provide a pharmaceutical composition comprising linagliptin in combination with an SGLT2 inhibitor which shows no signs or only marginal signs of degradation of linagliptin and thus enables a good to very good shelf life.

Another aim of the invention is to provide a pharmaceutical composition comprising linagliptin in combination with a SGLT2 inhibitor which has high content uniformity and/or which allows an effective production with regard to time and costs of pharmaceutical dosage forms.

Another aim of the invention is to provide a pharmaceutical dosage form comprising linagliptin in combination with an SGLT2 inhibitor which has a good shelf life, which has a short disintegration time, which has good dissolution properties and/or which enables a high bioavailability of linagliptin in a patient.

Another aim of the invention it to provide a pharmaceutical composition and a pharmaceutical dosage form, each comprising linagliptin in combination with an SGLT2 inhibitor, and a method for preventing, slowing progression of, delaying or treating a metabolic disorder, in particular of type 2 diabetes mellitus.

A further aim of the present invention is to provide a pharmaceutical composition and a pharmaceutical dosage form, each comprising linagliptin in combination with an SGLT2 inhibitor, and a method for improving glycemic control in a patient in need thereof, in particular in patients with type 2 diabetes mellitus.

Another aim of the present invention is to provide a pharmaceutical composition and a pharmaceutical dosage form, each comprising linagliptin in combination with an SGLT2 inhibitor, and a method for improving glycemic control in a patient with insufficient glycemic control despite monotherapy with an antidiabetic drug, for example metformin or an SGLT2 inhibitor or a DPPIV inhibitor.

Another aim of the present invention is to provide a pharmaceutical composition and a pharmaceutical dosage form, each comprising linagliptin in combination with an SGLT2 inhibitor, and a method for preventing, slowing or delaying progression from impaired glucose tolerance (IGT), impaired fasting blood glucose (IFG), insulin resistance and/or metabolic syndrome to type 2 diabetes mellitus.

Yet another aim of the present invention is to provide a pharmaceutical composition and a pharmaceutical dosage form, each comprising linagliptin in combination with an SGLT2 inhibitor, and a method for preventing, slowing progression of, delaying or treating of a condition or disorder from the group consisting of complications of diabetes mellitus.

A further aim of the present invention is to provide a pharmaceutical composition and a pharmaceutical dosage form, each comprising linagliptin in combination with an SGLT2 inhibitor, and a method for reducing the weight or preventing an increase of the weight in a patient in need thereof.

Another aim of the present invention is to provide a new pharmaceutical composition and a pharmaceutical dosage form, each comprising linagliptin in combination with an SGLT2 inhibitor, with a high efficacy for the treatment of metabolic disorders, in particular of diabetes mellitus, impaired glucose tolerance (IGT), impaired fasting blood glucose (IFG), and/or hyperglycemia, which has good to very good pharmacological and/or pharmacokinetic and/or physicochemical properties.

Another aim of the present invention is to provide a process for the preparation of a pharmaceutical dosage form according to the invention which is highly effective in costs and/or time.

Further aims of the present invention become apparent to the one skilled in the art by the description hereinbefore and in the following and by the examples.

**Summary of the Invention**

In a first aspect the present invention provides a pharmaceutical composition comprising linagliptin as a first active pharmaceutical ingredient and one or more excipients, in particular one or more diluents, one or more binders and/or one or more disintegrants. The pharmaceutical composition according to the invention is preferably a solid pharmaceutical composition, for example a solid pharmaceutical composition for oral administration.

Within the scope of the present invention it has been found that a pharmaceutical composition comprising linagliptin as an active pharmaceutical ingredient with a particle size distribution of X90 < 200 μm shows an advantageous dissolution profile and/or good bioavailability and allows a high content uniformity and an effective production with regard to time and costs of pharmaceutical dosage forms.

Therefore in another aspect the present invention provides a pharmaceutical composition comprising linagliptin as a first active pharmaceutical ingredient and one or more excipients, wherein the first active ingredient has a particle size distribution of X90 < 200 μm, preferably determined by volume by laser-diffraction method.

Furthermore within the scope of the present invention it has been found that linagliptin combined with certain excipients shows no signs or only marginal signs of degradation of linagliptin and thus enables a good to very good shelf life. In particular it has been found that the aims of the present invention can be achieved with a pharmaceutical composition as described above which comprises only one diluent.

Furthermore within the scope of the present invention it has been found that a pharmaceutical composition comprising linagliptin as a first active pharmaceutical ingredient in combination with a glucopyranosyl-substituted benzene derivative of the formula (I) as described hereinafter as an SGLT2 inhibitor shows no signs or only marginal signs of degradation of linagliptin and thus enables a good to very good shelf life. This result could not have been predicted in view of the chemical nature of linagliptin and the functional groups of the glucopyranosyl-substituted benzene derivative, in particular the glucopyranosyl-ring and the hydroxy-groups therein.

Therefore in another aspect the present invention provides a pharmaceutical composition comprising linagliptin as an active pharmaceutical ingredient, a glucopyranosyl-substituted benzene derivative of the formula (I)

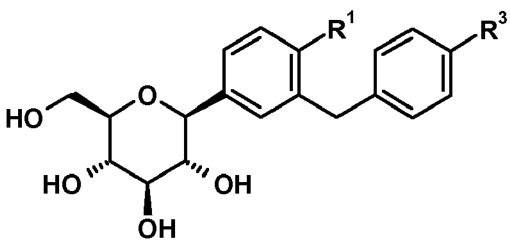

I

wherein $R^1$ denotes chloro or methyl; and $R^3$ denotes ethyl, ethynyl, ethoxy, *(R)*-tetrahydrofuran-3-yloxy or *(S)*-tetrahydrofuran-3-yloxy, or a prodrug thereof, as an active pharmaceutical ingredient, one or more diluents, one or more binders and one or more disintegrants.

Within the scope of the present invention it has been found that a pharmaceutical composition comprising the glucopyranosyl-substituted benzene derivative as an active pharmaceutical ingredient with a particle size distribution of 1 μm < X90 < 200 μm shows an advantageous dissolution profile and/or good bioavailability and allows a high content uniformity and an effective production with regard to time and costs of pharmaceutical dosage forms.

Therefore in another aspect the present invention provides a pharmaceutical composition comprising linagliptin as a first active pharmaceutical ingredient and a glucopyranosyl-substituted benzene derivative of the formula (I) as described hereinafter as a second active pharmaceutical ingredient and one or more excipients, wherein the second active ingredient has a particle size distribution of 1 μm < X90 < 200 μm, preferably determined by volume by laser-diffraction method.

The pharmaceutical compositions according to the invention allow a high content uniformity and an effective production with regard to time and costs of pharmaceutical dosage forms, such as tablets and capsules. Furthermore these pharmaceutical dosage forms, in particular tablets, such as one-layer tablets or two-layer tablets, according to the invention show no signs or only marginal signs of degradation of linagliptin and thus enable a long shelf life.

Therefore in another aspect the present invention provides a pharmaceutical dosage form comprising a pharmaceutical composition according to the invention. The pharmaceutical dosage forms according to the invention are preferably solid pharmaceutical dosage forms, even more preferably solid pharmaceutical dosage forms for oral administration.

In another aspect the present invention provides a process for the preparation of a pharmaceutical dosage form according to the invention comprising one or more granulation processes wherein the one or two active pharmaceutical ingredients together with one or more excipients are granulated.

Furthermore it can be found that the pharmaceutical composition comprising linagliptin in combination with a glucopyranosyl-substituted benzene derivative of the formula (I) as described hereinafter can advantageously be used for preventing, slowing progression of, delaying or treating a metabolic disorder, in particular for improving glycemic control in patients, for example in patients with inadequate glycemic control with existing therapy with oral antidiabetics. This opens up new therapeutic possibilities in the treatment and prevention of type 2 diabetes mellitus, overweight, obesity, complications of diabetes mellitus and of neighboring disease states.

According to another aspect of the invention, there is provided a method for preventing, slowing the progression of, delaying or treating a metabolic disorder selected from the group consisting of type 1 diabetes mellitus, type 2 diabetes mellitus, impaired glucose tolerance (IGT), impaired fasting blood glucose (IFG), hyperglycemia, postprandial hyperglycemia, overweight, obesity and metabolic syndrome in a patient in need thereof characterized in that a pharmaceutical composition or pharmaceutical dosage form as defined hereinbefore and hereinafter is administered to the patient.

According to another aspect of the invention, there is provided a method for improving glycemic control and/or for reducing of fasting plasma glucose, of postprandial plasma glucose and/or of glycosylated hemoglobin HbA1c in a patient in need thereof characterized in that an a pharmaceutical composition or pharmaceutical dosage form as defined hereinbefore and hereinafter is administered to the patient.

The pharmaceutical composition and pharmaceutical dosage form according to this invention may also have valuable disease-modifying properties with respect to diseases or conditions related to impaired glucose tolerance (IGT), impaired fasting blood glucose (IFG), insulin resistance and/or metabolic syndrome.

According to another aspect of the invention, there is provided a method for preventing, slowing, delaying or reversing progression from impaired glucose tolerance (IGT), impaired fasting blood glucose (IFG), insulin resistance and/or from metabolic syndrome to type 2 diabetes mellitus in a patient in need thereof characterized in that a pharmaceutical composition or pharmaceutical dosage form as defined hereinbefore and hereinafter is administered to the patient.

As by the use of a pharmaceutical composition and pharmaceutical dosage form according to this invention, an improvement of the glycemic control in patients in need thereof is obtainable, also those conditions and/or diseases related to or caused by an increased blood glucose level may be treated.

According to another aspect of the invention, there is provided a method for preventing, slowing the progression of, delaying or treating of a condition or disorder selected from the group consisting of complications of diabetes mellitus such as cataracts and micro- and macrovascular diseases, such as nephropathy, retinopathy, neuropathy, tissue ischaemia, diabetic foot, arteriosclerosis, myocardial infarction, accute coronary syndrome, unstable angina pectoris, stable angina pectoris, stroke, peripheral arterial occlusive disease, cardiomyopathy, heart failure, heart rhythm disorders and vascular restenosis, in a patient in need thereof characterized in that a pharmaceutical composition or pharmaceutical dosage form as defined hereinbefore and hereinafter is administered to the patient. The term "tissue ischaemia" particularly comprises diabetic macroangiopathy, diabetic microangiopathy, impaired wound healing and diabetic ulcer. In particular one or more aspects of diabetic nephropathy such as hyperperfusion, proteinuria and albuminuria may be treated, their progression slowed or their onset delayed or prevented. The terms "micro- and macrovascular diseases" and "micro- and macrovascular complications" are used interchangeably in this application.

By the administration of a pharmaceutical composition and pharmaceutical dosage form according to this invention and due to the activity of the SGLT2 inhibitor excessive blood glucose levels are not converted to insoluble storage forms, like fat, but excreted through the urine of the patient. Therefore, no gain in weight or even a reduction in body weight is the result.

According to another aspect of the invention, there is provided a method for reducing body weight or preventing an increase in body weight or facilitating a reduction in body weight in a patient in need thereof characterized in that a pharmaceutical composition or pharmaceutical dosage form as defined hereinbefore and hereinafter is administered to the patient.

The pharmacological effect of the glucopyranosyl-substituted benzene derivative as an SGLT2 inhibitor in the pharmaceutical composition according to this invention is independent of insulin. Therefore, an improvement of the glycemic control is possible without an additional strain on the pancreatic beta cells. By an administration of a pharmaceutical composition or pharmaceutical dosage form according to this invention a beta-cell degeneration and a decline of beta-cell functionality such as for example apoptosis or necrosis of pancreatic beta cells can be delayed or prevented. Furthermore, the functionality of pancreatic cells can be improved or restored, and the number and size of pancreatic beta cells increased. It may be shown that the differentiation status and hyperplasia of pancreatic beta-cells disturbed by hyperglycemia can be normalized by treatment with a pharmaceutical composition or pharmaceutical dosage form according to this invention.

According to another aspect of the invention, there is provided a method for preventing, slowing, delaying or treating the degeneration of pancreatic beta cells and/or the decline of the functionality of pancreatic beta cells and/or for improving and/or restoring the functionality of pancreatic beta cells and/or restoring the functionality of pancreatic insulin secretion in a patient in need thereof characterized in that a pharmaceutical composition or pharmaceutical dosage form as defined hereinbefore and hereinafter is administered to the patient.

By the administration of a pharmaceutical composition and pharmaceutical dosage form according to the present invention, an abnormal accumulation of fat in the liver may be reduced or inhibited. Therefore, according to another aspect of the present invention, there is provided a method for preventing, slowing, delaying or treating diseases or conditions attributed to an abnormal accumulation of liver fat in a patient in need thereof characterized in that a pharmaceutical composition or pharmaceutical dosage form as defined hereinbefore and hereinafter is administered to the

patient. Diseases or conditions which are attributed to an abnormal accumulation of liver fat are particularly selected from the group consisting of general fatty liver, non-alcoholic fatty liver (NAFL), non-alcoholic steatohepatitis (NASH), hyperalimentation-induced fatty liver, diabetic fatty liver, alcoholic-induced fatty liver or toxic fatty liver.

As a result thereof, another aspect of the invention provides a method for maintaining and/or improving the insulin sensitivity and/or for treating or preventing hyperinsulinemia and/or insulin resistance in a patient in need thereof characterized in that a pharmaceutical composition or pharmaceutical dosage form as defined hereinbefore and hereinafter is administered to the patient.

According to another aspect of the invention there is provided the use of a pharmaceutical composition according to the invention for the manufacture of a medicament for

- preventing, slowing the progression of, delaying or treating a metabolic disorder selected from the group consisting of type 1 diabetes mellitus, type 2 diabetes mellitus, impaired glucose tolerance (IGT), impaired fasting blood glucose (IFG), hyperglycemia, postprandial hyperglycemia, overweight, obesity and metabolic syndrome; or
- improving glycemic control and/or for reducing of fasting plasma glucose, of postprandial plasma glucose and/or of glycosylated hemoglobin HbA1c; or
- preventing, slowing, delaying or reversing progression from impaired glucose tolerance (IGT), impaired fasting blood glucose (IFG), insulin resistance and/or from metabolic syndrome to type 2 diabetes mellitus; or
- preventing, slowing the progression of, delaying or treating of a condition or disorder selected from the group consisting of complications of diabetes mellitus such as cataracts and micro- and macrovascular diseases, such as nephropathy, retinopathy, neuropathy, tissue ischaemia, diabetic foot, arteriosclerosis, myocardial infarction, accute coronary syndrome, unstable angina pectoris, stable angina pectoris, stroke, peripheral arterial occlusive disease, cardiomyopathy, heart failure, heart rhythm disorders and vascular restenosis; or
- reducing body weight or preventing an increase in body weight or facilitating a reduction in body weight; or
- preventing, slowing, delaying or treating the degeneration of pancreatic beta cells and/or the decline of the functionality of pancreatic beta cells and/or for improving and/or restoring the functionality of pancreatic beta cells and/or restoring the functionality of pancreatic insulin secretion; or
- preventing, slowing, delaying or treating diseases or conditions attributed to an abnormal accumulation of liver fat; or
- maintaining and/or improving the insulin sensitivity and/or for treating or preventing hyperinsulinemia and/or insulin resistance;

in a patient in need thereof.

According to another aspect of the invention, there is provided the use of a pharmaceutical composition or of a pharmaceutical dosage form according to the present invention for the manufacture of a medicament for a therapeutic and preventive method as described hereinbefore and hereinafter.

**Definitions**

The term **"active ingredient" or "active pharmaceutical ingredient"** of a pharmaceutical composition or pharmaceutical dosage form according to the present invention means linagliptin and optionally a glucopyranosyl-substituted benzene derivative of the formula (I) as according to the present invention, in particular the compound (I.3).

The term **"body mass index"** or **"BMI"** of a human patient is defined as the weight in kilograms divided by the square of the height in meters, such that BMI has units of $kg/m^2$.

The term **"overweight"** is defined as the condition wherein the individual has a BMI greater than or 25 $kg/m^2$ and less than 30 $kg/m^2$. The terms "overweight" and "pre-obese" are used interchangeably.

The term **"obesity"** is defined as the condition wherein the individual has a BMI equal to or greater than 30 $kg/m^2$. According to a WHO definition the term obesity may be categorized as follows: the term "class I obesity" is the condition wherein the BMI is equal to or greater than 30 $kg/m^2$ but lower than 35 $kg/m^2$; the term "class II obesity" is the condition wherein the BMI is equal to or greater than 35 $kg/m^2$ but lower than 40 $kg/m^2$; the term "class III obesity" is the condition wherein the BMI is equal to or greater than 40 $kg/m^2$.

The term **"visceral obesity"** is defined as the condition wherein a waist-to-hip ratio of greater than or equal to 1.0 in men and 0.8 in women is measured. It defines the risk for insulin resistance and the development of pre-diabetes.

The term **"abdominal obesity"** is usually defined as the condition wherein the waist circumference is > 40 inches or 102 cm in men, and is > 35 inches or 94 cm in women. With regard to a Japanese ethnicity or Japanese patients abdominal obesity may be defined as waist circumference ≥ 85 cm in men and ≥ 90 cm in women (see e.g. investigating committee for the diagnosis of metabolic syndrome in Japan).

The term **"euglycemia"** is defined as the condition in which a subject has a fasting blood glucose concentration within the normal range, greater than 70 mg/dL (3.89
mmol/L) and less than 100 mg/dL (5.6 mmol/L). The word "fasting" has the usual meaning as a medical term.

The term **"hyperglycemia"** is defined as the condition in which a subject has a fasting blood glucose concentration above the normal range, greater than 100 mg/dL (5.6 mmol/L). The word "fasting" has the usual meaning as a medical term.

The term **"hypoglycemia"** is defined as the condition in which a subject has a blood glucose concentration below the normal range, in particular below 70 mg/dL (3.89 mmol/L).

The term **"postprandial hyperglycemia"** is defined as the condition in which a subject has a 2 hour postprandial blood glucose or serum glucose concentration greater than 200 mg/dL (11.11 mmol/L).

The term **"impaired fasting blood glucose"** or **"IFG"** is defined as the condition in which a subject has a fasting blood glucose concentration or fasting serum glucose concentration in a range from 100 to 125 mg/dl (i.e. from 5.6 to 6.9 mmol/l), in particular greater than 110 mg/dL and less than 126 mg/dl (7.00 mmol/L). A subject with "normal fasting glucose" has a fasting glucose concentration smaller than 100 mg/dl, i.e. smaller than 5.6 mmol/l.

The term **"impaired glucose tolerance"** or **"IGT"** is defined as the condition in which a subject has a 2 hour postprandial blood glucose or serum glucose concentration greater than 140 mg/dl (7.78 mmol/L) and less than 200 mg/dL (11.11 mmol/L). The abnormal glucose tolerance, i.e. the 2 hour postprandial blood glucose or serum glucose concentration can be measured as the blood sugar level in mg of glucose per dL of plasma 2 hours after taking 75 g of glucose after a fast. A subject with "normal glucose tolerance" has a 2 hour postprandial blood glucose or serum glucose concentration smaller than 140 mg/dl (7.78 mmol/L).

The term **"hyperinsulinemia"** is defined as the condition in which a subject with insulin resistance, with or without euglycemia, has fasting or postprandial serum or plasma insulin concentration elevated above that of normal, lean individuals without insulin resistance, having a waist-to-hip ratio < 1.0 (for men) or < 0.8 (for women).

The terms "insulin-sensitizing", "insulin resistance-improving" or "insulin resistance-lowering" are synonymous and used interchangeably.

The term **"insulin resistance"** is defined as a state in which circulating insulin levels in excess of the normal response to a glucose load are required to maintain the euglycemic state (Ford ES, et al. JAMA. (2002) 287:356-9). A method of determining insulin resistance is the euglycaemic-hyperinsulinaemic clamp test. The ratio of insulin to glucose is determined within the scope of a combined insulin-glucose infusion technique. There is found to be insulin resistance if the glucose absorption is below the 25th percentile of the background population investigated (WHO definition). Rather less laborious than the clamp test are so called minimal models in which, during an intravenous glucose tolerance test, the insulin and glucose concentrations in the blood are measured at fixed time intervals and from these the insulin resistance is calculated. With this method, it is not possible to distinguish between hepatic and peripheral insulin resistance.

Furthermore, insulin resistance, the response of a patient with insulin resistance to therapy, insulin sensitivity and hyperinsulinemia may be quantified by assessing the "homeostasis model assessment to insulin resistance (HOMA-IR)" score, a reliable indicator of insulin resistance (Katsuki A, et al. Diabetes Care 2001; 24: 362-5). Further reference is made to methods for the determination of the HOMA-index for insulin sensitivity (Matthews et al., Diabetologia 1985, 28: 412-19), of the ratio of intact proinsulin to insulin *(*Forst et al., Diabetes 2003, 52(Suppl.1): A459*)* and to an euglycemic clamp study. In addition, plasma adiponectin levels can be monitored as a potential surrogate of insulin sensitivity. The estimate of insulin resistance by the homeostasis assessment model (HOMA)-IR score is calculated with the formula (Galvin P, et al. Diabet Med 1992;9:921-8):

$$\text{HOMA-IR} = [\text{fasting serum insulin } (\mu U/mL)] \times [\text{fasting plasma glucose}(mmol/L)/22.5]$$

As a rule, other parameters are used in everyday clinical practice to assess insulin resistance. Preferably, the patient's triglyceride concentration is used, for example, as increased triglyceride levels correlate significantly with the presence of insulin resistance.

Patients with a predisposition for the development of IGT or IFG or type 2 diabetes are those having euglycemia with hyperinsulinemia and are by definition, insulin resistant. A typical patient with insulin resistance is usually overweight or obese. If insulin resistance can be detected, this is a particularly strong indication of the presence of pre-diabetes. Thus, it may be that in order to maintain glucose homoeostasis a person needs 2-3 times as much insulin as a healthy person, without this resulting in any clinical symptoms.

The methods to investigate the **function of pancreatic beta-cells** are similar to the above methods with regard to insulin sensitivity, hyperinsulinemia or insulin resistance: An improvement of beta-cell function can be measured for example by determining a HOMA-index for beta-cell function (Matthews et al., Diabetologia 1985, 28: 412-19), the ratio of intact proinsulin to insulin *(*Forst et al., Diabetes 2003, 52(Suppl.1): A459*),* the insulin/C-peptide secretion after an oral glucose tolerance test or a meal tolerance test, or by employing a hyperglycemic clamp study and/or minimal

modeling after a frequently sampled intravenous glucose tolerance test *(*Stumvoll et al., Eur J Clin Invest 2001, 31: 380-81*)*.

The term **"pre-diabetes"** is the condition wherein an individual is pre-disposed to the development of type 2 diabetes. Pre-diabetes extends the definition of impaired glucose tolerance to include individuals with a fasting blood glucose within the high normal range ≥ 100 mg/dL (J. B. Meigs, et al. Diabetes 2003; 52:1475-1484) and fasting hyperinsulinemia (elevated plasma insulin concentration). The scientific and medical basis for identifying pre-diabetes as a serious health threat is laid out in a Position Statement entitled "The Prevention or Delay of Type 2 Diabetes" issued jointly by the American Diabetes Association and the National Institute of Diabetes and Digestive and Kidney Diseases (Diabetes Care 2002; 25:742-749).

Individuals likely to have insulin resistance are those who have two or more of the following attributes: 1) overweight or obese, 2) high blood pressure, 3) hyperlipidemia, 4) one or more 1$^{st}$ degree relative with a diagnosis of IGT or IFG or type 2 diabetes. Insulin resistance can be confirmed in these individuals by calculating the HOMA-IR score. For the purpose of this invention, insulin resistance is defined as the clinical condition in which an individual has a HOMA-IR score > 4.0 or a HOMA-IR score above the upper limit of normal as defined for the laboratory performing the glucose and insulin assays.

The term **"type 2 diabetes"** is defined as the condition in which a subject has a fasting blood glucose or serum glucose concentration greater than 125 mg/dL (6.94 mmol/L). The measurement of blood glucose values is a standard procedure in routine medical analysis. If a glucose tolerance test is carried out, the blood sugar level of a diabetic will be in excess of 200 mg of glucose per dL (11.1 mmol/l) of plasma 2 hours after 75 g of glucose have been taken on an empty stomach. In a glucose tolerance test 75 g of glucose are administered orally to the patient being tested after 10-12 hours of fasting and the blood sugar level is recorded immediately before taking the glucose and 1 and 2 hours after taking it. In a healthy subject, the blood sugar level before taking the glucose will be between 60 and 110 mg per dL of plasma, less than 200 mg per dL 1 hour after taking the glucose and less than 140 mg per dL after 2 hours. If after 2 hours the value is between 140 and 200 mg, this is regarded as abnormal glucose tolerance.

The term **"late stage type 2 diabetes mellitus"** includes patients with a secondary drug failure, indication for insulin therapy and progression to micro- and macrovascular complications e.g. diabetic nephropathy, or coronary heart disease (CHD).

The term **"HbA1c"** refers to the product of a non-enzymatic glycation of the haemoglobin B chain. Its determination is well known to one skilled in the art. In monitoring the treatment of diabetes mellitus the HbA1c value is of exceptional importance. As its production depends essentially on the blood sugar level and the life of the erythrocytes, the HbA1c in the sense of a "blood sugar memory" reflects the average blood sugar levels of the preceding 4-6 weeks. Diabetic patients whose HbA1c value is consistently well adjusted by intensive diabetes treatment (i.e. < 6.5 % of the total haemoglobin in the sample), are significantly better protected against diabetic microangiopathy. For example, metformin on its own achieves an average improvement in the HbA1c value in the diabetic of the order of 1.0 - 1.5 %. This reduction of the HbA1C value is not sufficient in all diabetics to achieve the desired target range of < 6.5 % and preferably < 6 % HbA1c.

The term **"insufficient glycemic control"** or "inadequate glycemic control" in the scope of the present invention means a condition wherein patients show HbA1c values above 6.5 %, in particular above 7.0 %, even more preferably above 7.5 %, especially above 8 %.

The **"metabolic syndrome",** also called "syndrome X" (when used in the context of a metabolic disorder), also called the "dysmetabolic syndrome" is a syndrome complex with the cardinal feature being insulin resistance (Laaksonen DE, et al. Am J Epidemiol 2002;156:1070-7). According to the ATP III/NCEP guidelines (Executive Summary of the Third Report of the National Cholesterol Education Program (NCEP) Expert Panel on Detection, Evaluation, and Treatment of High Blood Cholesterol in Adults (Adult Treatment Panel III) JAMA: Journal of the American Medical Association (2001) 285:2486-2497), diagnosis of the metabolic syndrome is made when three or more of the following risk factors are present:

1. Abdominal obesity, defined as waist circumference > 40 inches or 102 cm in men, and > 35 inches or 94 cm in women; or with regard to a Japanese ethnicity or Japanese patients defined as waist circumference ≥ 85 cm in men and ≥ 90 cm in women;
2. Triglycerides: ≥ 150 mg/dL
3. HDL-cholesterol < 40 mg/dL in men
4. Blood pressure ≥ 130/85 mm Hg (SBP ≥ 130 or DBP ≥ 85)
5. Fasting blood glucose ≥ 100 mg/dL

The NCEP definitions have been validated (Laaksonen DE, et al. Am J Epidemiol. (2002) 156:1070-7). Triglycerides and HDL cholesterol in the blood can also be determined by standard methods in medical analysis and are described for example in Thomas L (Editor): "Labor und Diagnose", TH-Books Verlagsgesellschaft mbH, Frankfurt/Main, 2000.

According to a commonly used definition, **hypertension** is diagnosed if the systolic blood pressure (SBP) exceeds a value of 140 mm Hg and diastolic blood pressure (DBP) exceeds a value of 90 mm Hg. If a patient is suffering from manifest diabetes it is currently recommended that the systolic blood pressure be reduced to a level below 130 mm Hg and the diastolic blood pressure be lowered to below 80 mm Hg.

The terms **"treatment"** and "treating" comprise therapeutic treatment of patients having already developed said condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and dosage forms and methods of the present invention may be used for instance as therapeutic treatment over a period of time as well as for chronic therapy.

The terms **"prophylactically treating",** "preventivally treating" and "preventing" are used interchangeably and comprise a treatment of patients at risk to develop a condition mentioned hereinbefore, thus reducing said risk.

The term **"therapeutically effective amount"** as used herein refers to an amount or dose of the active pharmaceutical ingredient that effects the desired therapeutic response, for example a reduction in blood glucose levels, a reduction of HbA1c or weight reduction, in a mammalian subject or patient, but preferably does not cause hypoglycemia in the subject or patient. In case the pharmaceutical composition or pharmaceutical dosage form comprises two active pharmaceutical ingredients, the term "therapeutically effective amount" as used herein refers to an amount or dose of the respective active pharmaceutical ingredient that in combination with the other active pharmaceutical ingredient effects the desired therapeutic response, for example a reduction in blood glucose levels, a reduction of HbA1c or weight reduction, in a mammalian subject or patient, but preferably does not cause hypoglycemia in the subject or patient.

The term **"tablet"** comprises tablets without a coating and tablets with one or more coatings. Furthermore the "term" tablet comprises tablets having one, two, three or even more layers and press-coated tablets, wherein each of the beforementioned types of tablets may be without or with one or more coatings. The term "tablet" also comprises mini, melt, chewable, effervescent and orally disintegrating tablets.

The terms **"pharmacopoe"** and **"pharmacopoeias"** refer to standard pharmacopoeias such as the "USP 31-NF 26 through Second Supplement" (United States Pharmacopeial Convention) or the "European Pharmacopoeia 6.3" (European Directorate for the Quality of Medicines and Health Care, 2000-2009).

## Brief Description of the Figures

The Figure 1 shows an X-ray powder diffractogram of the crystalline form (I.3X) of the compound (I.3).

The Figure 2 shows the thermoanalysis and determination of the melting point via DSC of the crystalline form (I3.X) of the compound (I.3).

The Figure 3 shows the glucose excursion as quantified by the calculated reactive glucose AUC after a glucose challenge in four different groups of ZDF rats which received a control, linagliptin (Cpd. A), the compound (I.3) (Cpd. B) or a combination of linagliptin and the compound (I.3) (Combination A + B).

The Figure 4 shows dissolution profiles of tablets according to the Example 4 and the Example 6 wherein API 1 is the compound (I.3) and the API 2 is linagliptin.

The Figure 5 shows dissolution profiles of tablets according to the Example 8 wherein API 1 is the compound (I.3) and the API 2 is linagliptin.

## Detailed Description

The aspects according to the present invention, in particular the pharmaceutical compositions, pharmaceutical dosage forms, methods and uses, refer to linagliptin and glucopyranosyl-substituted benzene derivatives as defined hereinbefore and hereinafter.

The term "linagliptin" as employed herein refers to linagliptin and pharmaceutically acceptable salts thereof, including hydrates and solvates thereof, and crystalline forms thereof. Crystalline forms are described in WO 2007/128721. Preferred crystalline forms are the polymorphs A and B described therein. Methods for the manufacture of linagliptin are described in the patent applications WO 2004/018468 and WO 2006/048427 for example. Linagliptin is distinguished from structurally comparable DPP IV inhibitors, as it combines exceptional potency and a long-lasting effect with favourable pharmacological properties, receptor selectivity and a favourable side-effect profile or bring about unexpected therapeutic advantages or improvements when used in combination with a glucopyranosyl-substituted benzene derivative according to this invention.

The glucopyranosyl-substituted benzene derivative is defined by the formula (I)

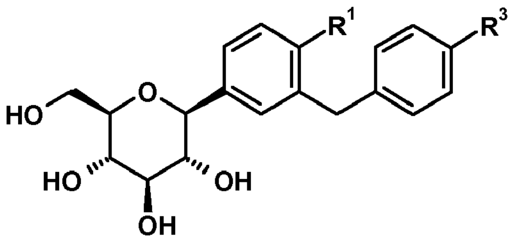

I

wherein $R^1$ denotes chloro or methyl; and $R^3$ denotes ethyl, ethynyl, ethoxy, *(R)*-tetrahydrofuran-3-yloxy or *(S)*-tetrahydrofuran-3-yloxy; or a prodrug thereof.

Compounds of the formula (I) and methods of their synthesis are described for example in the following patent applications: WO 2005/092877, WO 2006/117360, WO 2006/117359,

WO 2006/120208, WO 2006/064033, WO 2007/028814, WO 2007/031548, WO 2008/049923.

In the above glucopyranosyl-substituted benzene derivatives of the formula (I) the following definitions of the substituents are preferred.

Preferably $R^1$ denotes chloro.

Preferably $R^3$ denotes ethynyl, *(R)*-tetrahydrofuran-3-yloxy or *(S)*-tetrahydrofuran-3-yloxy. Most preferably $R^3$ denotes *(R)*-tetrahydrofuran-3-yloxy or *(S)*-tetrahydrofuran-3-yloxy.

Preferred glucopyranosyl-substituted benzene derivatives of the formula (I) are selected from the group of compounds (I.1) to (I.5):

| | |
|---|---|
| (I.1) | 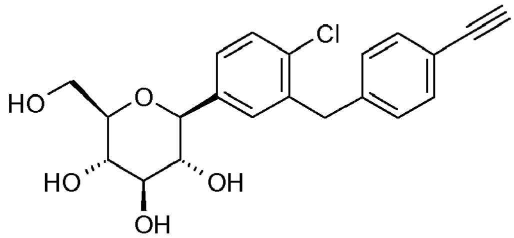 1-chloro-4-(β-D-glucopyranos-1-yl)-2-(4-ethynyl-benzyl)-benzene, |
| (I-2) | 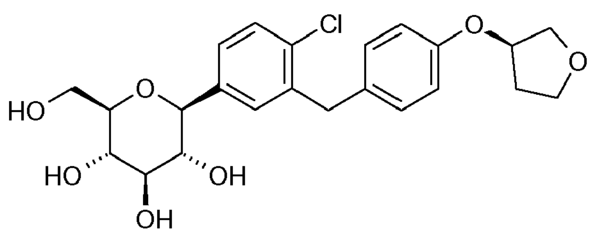 1-chloro-4-(β-D-glucopyranos-1-yl)-2-[4-((*R*)-tetrahydrofuran-3-yloxy)-benzyl]-benzene, |
| (I.3) | 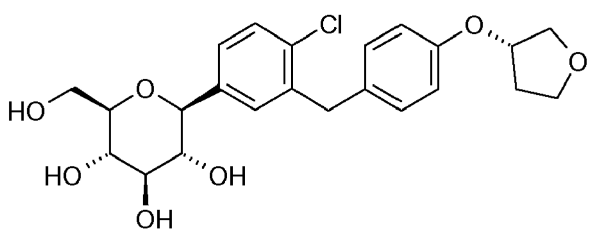 1-chloro-4-(β-D-glucopyranos-1-yl)-2-[4-((*S*)-tetrahydrofuran-3-yloxy)-benzyl]-benzene, |

(continued)

| | |
|---|---|
| (I-4) | 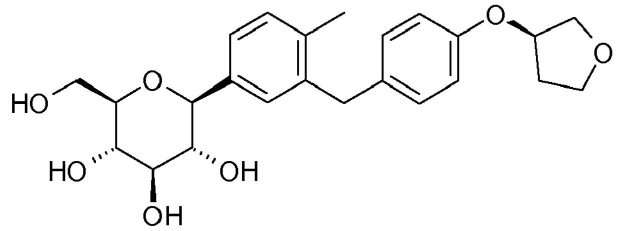 1-methyl-2-[4-((*R*)-tetrahydrofuran-3-yloxy)-benzyl]-4-(β-D-glucopyranos-1-yl)-benzene, |
| (I-5) | 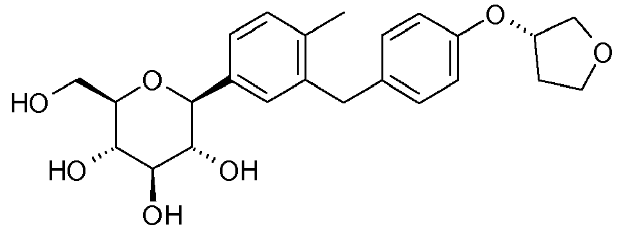 1-methyl-2-[4-((*S*)-tetrahydrofuran-3-yloxy)-benzyl]-4-(β-D-glucopyranos-1-yl)-benzene. |

Even more preferred glucopyranosyl-substituted benzene derivatives of the formula (I) are selected from the compounds (I.2) and (I.3).

According to this invention, it is to be understood that the definitions of the above listed glucopyranosyl-substituted benzene derivatives of the formula (I) also comprise their hydrates, solvates and polymorphic forms thereof, and prodrugs thereof. With regard to the preferred compound (I.1) an advantageous crystalline form is described in the international patent application WO 2007/028814 which hereby is incorporated herein in its entirety. With regard to the preferred compound (I.2), an advantageous crystalline form is described in the international patent application WO 2006/117360 which hereby is incorporated herein in its entirety. With regard to the preferred compound (I.3) an advantageous crystalline form is described in the international patent application WO 2006/117359 which hereby is incorporated herein in its entirety. With regard to the preferred compound (I.5) an advantageous crystalline form is described in the international patent application WO 2008/049923 which hereby is incorporated herein in its entirety. These crystalline forms possess good solubility properties which enable a good bioavailability of the glucopyranosyl-substituted benzene derivative. Furthermore, the crystalline forms are physico-chemically stable and thus provide a good shelf-life stability of the pharmaceutical composition.

A preferred crystalline form (I.3X) of the compound (I.3) can be characterized by an X-ray powder diffraction pattern that comprises peaks at 18.84, 20.36 and 25.21 degrees 20 (±0.1 degrees 20), wherein said X-ray powder diffraction pattern (XRPD) is made using $CuK_{\alpha 1}$ radiation.

In particular said X-ray powder diffraction pattern comprises peaks at 14.69, 18.84, 19.16, 19.50, 20.36 and 25.21 degrees 20 (±0.1 degrees 20), wherein said X-ray powder diffraction pattern is made using $CuK_{\alpha 1}$ radiation.

In particular said X-ray powder diffraction pattern comprises peaks at 14.69, 17.95, 18.43, 18.84, 19.16, 19.50, 20.36, 22.71, 23.44, 24.81, 25.21 and 25.65 degrees 20 (±0.1 degrees 20), wherein said X-ray powder diffraction pattern is made using $CuK_{\alpha 1}$ radiation.

More specifically, the crystalline form (I.3X) is characterised by an X-ray powder diffraction pattern, made using $CuK_{\alpha 1}$ radiation, which comprises peaks at degrees 20 (±0.1 degrees 20) as contained in Table 1.

**Table 1:** X-ray powder diffraction pattern of the crystalline form (I.3X) (only peaks up to 30° in 2 Θ are listed):

| **2Θ [°]** | **d-value [Å]** | **Intensity $I/I_0$ [%]** |
|---|---|---|
| 4.46 | 19.80 | 8 |
| 9.83 | 8.99 | 4 |
| 11.68 | 7.57 | 4 |
| 13.35 | 6.63 | 14 |
| 14.69 | 6.03 | 42 |
| 15.73 | 5.63 | 16 |

(continued)

| 2Θ [°] | d-value [Å] | Intensity $I/I_0$ [%] |
|---|---|---|
| 16.20 | 5.47 | 8 |
| 17.95 | 4.94 | 30 |
| 18.31 | 4.84 | 22 |
| 18.43 | 4.81 | 23 |
| 18.84 | 4.71 | 100 |
| 19.16 | 4.63 | 42 |
| 19.50 | 4.55 | 31 |
| 20.36 | 4.36 | 74 |
| 20.55 | 4.32 | 13 |
| 21.18 | 4.19 | 11 |
| 21.46 | 4.14 | 13 |
| 22.09 | 4.02 | 19 |
| 22.22 | 4.00 | 4 |
| 22.71 | 3.91 | 28 |
| 23.44 | 3.79 | 27 |
| 23.72 | 3.75 | 3 |
| 24.09 | 3.69 | 3 |
| 24.33 | 3.66 | 7 |
| 24.81 | 3.59 | 24 |
| 25.21 | 3.53 | 46 |
| 25.65 | 3.47 | 23 |
| 26.40 | 3.37 | 2 |
| 26.85 | 3.32 | 8 |
| 27.26 | 3.27 | 17 |
| 27.89 | 3.20 | 2 |
| 28.24 | 3.16 | 3 |
| 29.01 | 3.08 | 4 |
| 29.41 | 3.03 | 18 |

Even more specifically, the crystalline form (I.3X) is characterised by an X-ray powder diffraction pattern, made using $CuK_{\alpha 1}$ radiation, which comprises peaks at degrees 2Θ (±0.1 degrees 2Θ) as shown in Figure 1.

Furthermore the crystalline form (I.3X) is characterised by a melting point of about 149°C ± 3°C (determined via DSC; evaluated as onset-temperature; heating rate 10 K/min).The obtained DSC curve is shown in Figure 2.

The X-ray powder diffraction patterns are recorded, within the scope of the present invention, using a STOE - STADI P-diffractometer in transmission mode fitted with a location-sensitive detector (OED) and a Cu-anode as X-ray source (CuKα1 radiation, λ = 1,54056 Å, 40kV, 40mA). In the Table 1 above the values "2Θ [°]" denote the angle of diffraction in degrees and the values "d [Å]" denote the specified distances in Å between the lattice planes. The intensity shown in the Figure 1 is given in units of cps (counts per second).

In order to allow for experimental error, the above described 2Θ values should be considered accurate to ± 0.1 degrees 2Θ, in particular ± 0.05 degrees 2Θ. That is to say, when assessing whether a given sample of crystals of the compound (I.3) is the crystalline form in accordance with the invention, a 2Θ value which is experimentally observed for the sample should be considered identical with a characteristic value described above if it falls within ± 0.1 degrees 2Θ

of the characteristic value, in particular if it falls within ± 0.05 degrees 2Θ of the characteristic value.

The melting point is determined by DSC (Differential Scanning Calorimetry) using a DSC 821 (Mettler Toledo).

Regarding the active pharmaceutical ingredients it can be found that the dissolution properties of the pharmaceutical composition and dosage form and thus the bioavailability of the active ingredients is affected *inter alia* by the particle size and particle size distribution of the respective active pharmaceutical ingredient. In the pharmaceutical composition and pharmaceutical dosage form according to the invention the active pharmaceutical ingredients preferably have a particle size distribution such that at least 90 % of the respective active pharmaceutical ingredient particles, with regard to the distribution by volume, has a particle size smaller than 200 μm, i.e. X90 < 200 μm.

In particular in the pharmaceutical composition and pharmaceutical dosage form according to the invention linagliptin, for example a crystalline form thereof, preferably has a particle size distribution (by volume) such that at least 90 % of the respective active pharmaceutical ingredient has a particle size smaller than 200 μm, i.e. X90 < 200 μm, more preferably X90 ≤ 150 μm. More preferably the particle size distribution is such that X90 ≤ 100 μm, even more preferably X90 ≤ 75 μm. In addition the particle size distribution is preferably such that X90 > 0.1 μm, more preferably X90 ≥ 1 μm, most preferably X90 ≥ 5 μm. Therefore preferred particle size distributions are such that 0.1 μm < X90 < 200 μm, particularly 0.1 μm < X90 ≤ 150 μm, more preferably 1 μm ≤ X90 ≤ 150 μm, even more preferably 5 μm ≤ X90 ≤ 100 μm. A preferred example of a particle size distribution of linagliptin is such that X90 ≤ 50 μm or 10 μm ≤ X90 ≤ 50 μm.

Furthermore in the pharmaceutical composition and pharmaceutical dosage form according to the invention linagliptin, for example a crystalline form thereof, preferably has a particle size distribution (by volume) such that X50 ≤ 90 μm, more preferably X50 ≤ 75 μm, even more preferably X50 ≤ 50 μm, most preferably X50 ≤ 40 μm. In addition the particle size distribution is preferably such that X50 ≥ 0.1 μm, more preferably X50 ≥ 0.5 μm, even more preferably X50 ≥ 4 μm. Therefore preferred particle size distributions are such that 0.1 μm ≤ X50 ≤ 90 μm, particularly 0.5 μm ≤ X50 ≤ 75 μm, more preferably 4 μm ≤ X50 ≤ 75 μm, even more preferably 4 μm ≤ X50 ≤ 50 μm. A preferred example is 8 μm ≤ X50 ≤ 40 μm.

Furthermore in the pharmaceutical composition and pharmaceutical dosage form according to the invention linagliptin, for example a crystalline form thereof, preferably has a particle size distribution (by volume) such that X10 ≥ 0.05 μm, more preferably X10 ≥ 0.1 μm, even more preferably X10 ≥ 0.5 μm.

In particular with regard to the glucopyranosyl-substituted benzene derivative of the formula (I), in particular the compound (I.3), it is surprisingly found that too small particle sizes influence the manufacturability, for example by sticking or filming. On the other hand too large particles negatively affect the dissolution properties of the pharmaceutical composition and dosage form and thus the bioavailability. In the following preferred ranges of the particle size distribution are described.

In the pharmaceutical composition and pharmaceutical dosage form according to the invention the glucopyranosyl-substituted benzene derivative of the formula (I), in particular the compound (I.3), for example its crystalline form (I3.X), preferably has a particle size distribution (by volume) such that at least 90 % of the respective active pharmaceutical ingredient has a particle size smaller than 200 μm, i.e. X90 < 200 μm, preferably X90 ≤ 150 μm. More preferably the particle size distribution is such that X90 ≤ 100 μm, even more preferably X90 ≤ 90 μm. In addition the particle size distribution is preferably such that X90 ≥ 1 μm, more preferably X90 ≥ 5 μm, even more preferably X90 ≥ 10 μm. Therefore preferred particle size distributions are such that 1 μm ≤ X90 < 200 μm, particularly 1 μm ≤ X90 ≤ 150 μm, more preferably 5 μm ≤ X90 ≤ 150 μm, even more preferably 5 μm ≤ X90 ≤ 100 μm, even more preferably 10 μm ≤ X90 ≤ 100 μm. A preferred example X90 ≤ 75 μm. Another preferred example is 20 μm ≤ X90 ≤ 50 μm.

Furthermore in the pharmaceutical composition and pharmaceutical dosage form according to the invention the glucopyranosyl-substituted benzene derivative of the formula (I), in particular the compound (I.3), for example its crystalline form (I3.X), preferably has a particle size distribution (by volume) such that X50 ≤ 90 μm, more preferably X50 ≤ 75 μm, even more preferably X50 ≤ 50 μm, most preferably X50 ≤ 40 μm. In addition the particle size distribution is preferably such that X50 ≥ 1 μm, more preferably X50 ≥ 5 μm, even more preferably X50 ≥ 8 μm. Therefore preferred particle size distributions are such that 1 μm X50 ≤ 90 μm, particularly 1 μm ≤ X50 ≤ 75 μm, more preferably 5 μm ≤ X50 ≤ 75 μm, even more preferably 5 μm ≤ X50 ≤ 50 μm. A preferred example is 8 μm ≤ X50 ≤ 40 μm.

Furthermore in the pharmaceutical composition and pharmaceutical dosage form according to the invention the glucopyranosyl-substituted benzene derivative of the formula (I), in particular the compound (I.3), for example its crystalline form (I3.X), preferably has a particle size distribution (by volume) such that X10 ≥ 0.1 μm, more preferably X10 ≥ 0.5 μm, even more preferably X10 ≥ 1 μm.

Therefore a pharmaceutical composition or pharmaceutical dosage form according to this invention may preferably be characterized by the above specified particle size distributions X90, X50 and/or X10 or one of the following embodiments:

| Embodiment | Linagliptin | Glucopyranosyl-substituted benzene derivative, in particular of the compound (I.3) |
|---|---|---|
| E.1 | X90 < 200 μm | X90 < 200 μm |
| E.2 | 0.1 μm ≤ X90 ≤ 150 μm | 1 μm ≤ X90 ≤ 150 μm |
| E.3 | 0.1 μm ≤ X90 ≤ 150 μm | 5 μm ≤ X90 ≤ 150 μm |
| E.4 | 0.1 μm ≤ X90 ≤ 150 μm | 10 μm ≤ X90 ≤ 100 μm |
| E.5 | 0.1 μm ≤ X90 ≤ 150 μm | X90 ≤ 150 μm<br>1 μm ≤ X50 ≤ 75 μm |
| E.6 | 0.1 μm ≤ X90 ≤ 150 μm | X90 ≤ 150 μm<br>5 μm ≤ X50 ≤ 50 μm |
| E.7 | 0.1 μm ≤ X90 ≤ 150 μm | X90 ≤ 150 μm<br>1 μm ≤ X50 ≤ 75 μm<br>X10 ≥ 0.1 μm |
| E.8 | 0.1 μm ≤ X90 ≤ 150 μm | X90 ≤ 150 μm<br>5 μm ≤ X50 ≤ 50μm<br>X10 ≥ 0.5 μm |
| E.9 | 0.1 μm ≤ X90 ≤ 150 μm | X90 ≤ 100 μm<br>5 μm ≤ X50 ≤ 50μm<br>X10 ≥ 0.5 μm |
| E.10 | 5 μm ≤ X90 ≤ 100 μm | X90 ≤ 100 μm<br>5 μm ≤ X50 ≤ 50μm<br>X10 ≥ 0.5 μm |
| E.11 | X90 ≤ 150 μm<br>4 μm ≤ X50 ≤ 75 μm | X90 ≤ 100 μm<br>5 μm ≤ X50 ≤ 50μm<br>X10 ≥ 0.5 μm |
| E.12 | X90 ≤ 100 μm<br>4 μm ≤ X50 ≤ 75 μm<br>X10 ≥ 0.05 μm | X90 ≤ 100 μm<br>5 μm ≤ X50 ≤ 50μm<br>X10 ≥ 0.5 μm |
| E.13 | X90 ≤ 100 μm<br>4 μm ≤ X50 ≤ 50 μm<br>X10 ≥ 0.1 μm | X90 ≤ 100 μm<br>5 μm ≤ X50 ≤ 50μm<br>X10 ≥ 0.5 μm |

The value X90 refers to the 90% value of the volume distribution measured using a laser diffractometer. In other words, for the purposes of the present invention, the X90 value denotes the particle size below which 90% of the quantity of particles is found based on the volume distribution. Analogously the value X50 refers to the 50% value (median) of the volume distribution measured using a laser diffractometer. In other words, for the purposes of the present invention, the X50 value denotes the particle size below which 50% of the quantity of particles is found based on the volume distribution. Analogously the value X10 refers to the 10% value of the volume distribution measured using a laser diffractometer. In other words, for the purposes of the present invention, the X10 value denotes the particle size below which 10% of the quantity of particles is found based on the volume distribution.

Preferably all X90, X50, X10 values hereinbefore and hereinafter are by volume and determined by laser-diffraction method, in particular low angle laser light scattering, i.e. Fraunhofer diffraction. A preferred test is described in the experimental section. The laser diffraction method is sensitive to the volume of a particle and provides a volume-average particle size, which is equivalent to the weight-average particle size if the density is constant. The skilled artesian knows that the results of the particle size distribution determination by one technique can be correlated with that from another technique, for example on an empirical basis by routine experimentation. Alternatively the particle size distribution in the pharmaceutical composition or dosage form can be determined by microscopy, in particular electron microscopy or scanning electron microscopy.

In order to provide suitable starting material consisting the active pharmaceutical ingredient, such as linagliptin

or the glucopyranosyl-substituted benzene derivative, in particular the compound (I.3) and its crystalline form (I.3X), is milled, for example jet-milled or pin-milled.

In the following the preferred excipients and carriers in the pharmaceutical compositions according to the invention are described in further detail. Preferably the excipients are pharmaceutically acceptable.

Preferably the excipients are chosen such that they are compatible with linagliptin, i.e. such that there is no or only marginal degradation of linagliptin in the pharmaceutical composition. The degradation can be tested in standard tests, for example after a 6 months storage at 40°C and 75% relative humidity. In this context the term "marginal degradation" shall mean a chemical degradation of linagliptin of less than 5 %, preferably less than 3 %, even more preferably less than 2 % by weight of linagliptin. The content and thus the degradation can be determined by well-known analytical methods, for example using HPLC or UV methods.

In the pharmaceutical composition according to the invention the excipients preferably comprise one or more diluents.

Furthermore in the pharmaceutical composition according to the invention the excipients preferably comprise one or more diluents and one or more binders.

Furthermore in the pharmaceutical composition according to the invention the excipients preferably comprise one or more diluents and one or more binders and one or more disintegrants and optional further ingredients.

Furthermore in the pharmaceutical composition according to the invention the excipients even more preferably comprise one or more diluents and one or more binders and one or more disintegrants and one or more lubricants and optional further ingredients.

Some of the excipients may have two or more functions at the same time, for example may act as a diluent and as a binder or as a binder and as disintegrant or as a diluent, as a binder and as disintegrant.

The one or more diluents, another term is filler, are added as the quantity of the active pharmaceutical ingredient(s) is small and thus to achieve a minimal tablet weight (for example 100 mg or more) and a satisfying content uniformity (for example < 3 % standard deviation) according to the pharmacopeias. Common diluents as for example lactose, sucrose, and microcrystalline cellulose are observed as not being compatible with linagliptin.

Preferably the one or more diluents suitable for a pharmaceutical composition according to the invention are selected from the group consisting of cellulose, in particular cellulose powder, dibasic calciumphosphate, in particular anhydrous or dibasic calciumphosphate dihydrate, erythritol, mannitol, starch, pregelatinized starch, and xylitol, including derivatives and hydrates of the beforementioned substances. The diluents pre-gelatinized starch shows additional binder properties. Among the diluents listed above mannitol and pregelatinized starch are particularly preferred.

In case the pharmaceutical composition according to the invention comprises one diluent, then the diluent is preferably mannitol or pregelatinized starch, most preferably mannitol.

In case the pharmaceutical composition according to the invention comprises two or more diluents, then the first diluent is preferably mannitol and the second diluent is selected from the group of diluents as described hereinbefore, even more preferably pregelatinized starch which shows additional binder properties.

Mannitol as mentioned hereinbefore and hereinafter is preferably a grade with small particle size suitable for granulation. An example is Pearlitol™ 50C (Roquette).

Pregelatinized starch as mentioned hereinbefore and hereinafter can be any of the commercially available grades. An example is Starch 1500™ (Colorcon).

The pharmaceutical composition according to the present invention preferably does not comprise a substance selected from the group glucose, fructose, sucrose, lactose and maltodextrines, in particular lactose. Preferably it does not comprise a substance of the beforementioned group, in particular lactose, above an amount of 2 % by weight of the total composition, even more preferably above an amount of 0.5 % by weight of the total composition.

The one or more binders in the pharmaceutical composition provide adhesiveness to the pharmaceutical composition, for example during the granulation, and to the compressed tablet. They add to the cohesive strength already available in the diluent. Common binders are for example sucrose and microcrystalline cellulose which were observed as not being compatible with linagliptin.

Preferably the one or more binders suitable for a pharmaceutical composition according to the invention are selected from the group consisting of copovidone, hydroxypropyl methylcellulose (HPMC), hydroxypropylcellulose (HPC) and a polyvinylpyrrolidone, pregelatinized starch, and low-substituted hydroxypropylcellulose (L-HPC), including derivatives and hydrates of the beforementioned substances. An even more preferred binder is copovidone and/or pregelatinized starch.

Copovidone as mentioned hereinbefore and hereinafter is preferably a copolymerisate of vinylpyrrolidon with vinyl acetate, preferably with a molecular weight from about 45000 to about 70000. An example is Kollidon™ VA 64 (BASF).

Hydroxypropyl methylcellulose (also called HPMC or hypromellose) as mentioned hereinbefore and hereinafter is preferably hypromellose 2910. Hydroxypropyl methylcellulose has preferably a visicosity in the range from about 4 to about 6 cps. An example is Methocel™ E5 Prem LV (Dow Chemicals).

Hydroxypropyl cellulose (also called HPC) as mentioned hereinbefore and hereinafter has preferably a viscosity range in the range from about 300 to about 600 mPa*s. Hydroxypropyl cellulose has preferably a molecular weight from about 60000 to about 100000, for example around 80000. An example is Klucel™ EF (Aqualon).

Polyvinylpyrrolidone (also called PVP, polyvidone or povidone) as mentioned hereinbefore and hereinafter has preferably a molecular weight from about 28000 to about 54000. Polyvinylpyrrolidone has preferably a viscosity range from about 3.5 to about 8.5 mPa*s. An example is Kollidon™ 25 or Kollidon™ 30 (BASF).

Low-substituted hydroxypropylcellulose (also called L-HPC) as mentioned hereinbefore and hereinafter has preferably a hydroxypropoxy content in a range from about 5 to about 16 % by weight.

The above mentioned binders pregelatinized starch and L-HPC show additional diluent and disintegrant properties and can also be used as the second diluent or the disintegrant.

The one or more disintegrating agents serves to assist in the fragmentation of the pharmaceutical composition and dosage form after administration. A common disintegrant is for example microcrystalline cellulose which was observed as not being compatible with linagliptin.

Preferably the one or more disintegrants suitable for a pharmaceutical composition according to the present invention are selected from the group consisting of crospovidone, low-substituted hydroxypropylcellulose (L-HPC), and starches, such as native starches, in particular corn starch, and pregelatinized starch, including derivatives and hydrates of the beforementioned substances. Among the beforementioned disintegrants corn starch, pregelatinized starch and crospovidone are even more preferred.

Surprisingly it has been found that at least two disintegrants are preferred, if linagliptin and a glucopyranosyl-substituted benzene derivative of the formula (I) are combined in a pharmaceutical composition according to the invention, in particular in one dosage form, e.g. a tablet or a capsule. Preferred disintegrants are corn starch and crospovidone.

Even more preferred is a combination of at least three disintegrants, if linagliptin and a glucopyranosyl-substituted benzene derivative of the formula (I) inhibitor are combined in a pharmaceutical composition according to the invention, in particular in one dosage form, e.g. a tablet or a capsule. Preferred disintegrants are corn starch, pregelatinized starch and crospovidone.

Crospovidone as mentioned hereinbefore and hereinafter is preferably an insoluble polyvidone, i.e. a cross-linked form of PVP. An example is Kollidon™ CL or Kollidon™ CL-SF (BASF).

Corn starch as mentioned hereinbefore and hereinafter is preferably a native starch. An example is Maize starch (extra white) (Roquette).

The above mentioned disintegrants starch and pregelatinized starch show additional diluent properties, and thus can also be used as the second diluent for example.

The one or more lubricants in the pharmaceutical composition reduce friction in the preparation of the tablet, i.e. during the compression and ejection cycle. In addition, they aid in preventing adherence of tablet material to the dies and punches.

Preferably the pharmaceutical composition according to the present invention additionally comprises one or more lubricants. Preferably the one or more lubricants suitable for a pharmaceutical composition according to the invention are selected from the group consisting of talc (e.g. from Luzenac), polyethylene glycol, in particular polyethylene glycol with a molecular weight in a range from about 4400 to about 9000, hydrogenated castor oil, fatty acid and salts of fatty acids, in particular the calcium, magnesium, sodium or potassium salts thereof, for example calcium behenate, calcium stearate, sodium stearyl fumarate or magnesium stearate (for example (e.g. HyQual®, Mallinckrodt or Ligamed®, Peter Greven). More preferred lubricants are magnesium stearate and talc.

Surprisingly it has been found that at least two lubricants are preferred, if linagliptin and a glucopyranosyl-substituted benzene derivative of the formula (I) are combined in a pharmaceutical composition according to the invention, in particular in one dosage form, e.g. a tablet or a capsule. Preferred lubricants are talc and magnesium stearate. The combination of the two or more lubricants enables low ejection forces and avoids sticking of the final blend in the manufacture of tablets for example.

The one or more glidants are agents that improve powder fluidity in the pharmaceutical composition.

The pharmaceutical composition according to the present invention may additionally comprise one or more glidants. Preferably the one or more glidants suitable for a pharmaceutical composition according to the invention are selected from the group consisting of talc and colloidal silicon dioxide (e.g. Aerosil™ 200 Pharma (Evonik)).

It is preferred that the excipients, in particular the one or more diluents, such as mannitol, have a particle size in the range from 1 to 500 μm. A particle size from 25 to 160 μm is preferred in granulation processes. A particle size from 180 to 500 μm is preferred in direct tabletting processes. The particle size is preferably analyzed via sieving. Preferably at least 80 %, more preferably at least 90 %, most preferably at least 95 % by weight of the particles is in the given range.

According to a first embodiment of the present invention the pharmaceutical composition comprises only one active pharmaceutical ingredient which is linagliptin.

A preferred composition according to the first embodiment of the present invention comprises a diluent, a binder

and a disintegrant. Preferably said composition comprises only one diluent. Even more preferably said composition comprises only one diluent and only one binder. Even more preferably said composition comprises only one diluent, only one binder and only one disintegrant. The composition may additionally comprise at least one lubricant. Furthermore the composition may additionally comprise at least one glidant.

A pharmaceutical composition according to the first embodiment comprises preferably

| | |
|---|---|
| 0.5-20 % | active pharmaceutical ingredient, |
| 40-88 % | one or more, preferably one diluent, |
| 0.5-20 % | one or more binders, |
| 0.5-20 % | one or more disintegrants, |

wherein the percentages are by weight of the total composition.

The following ranges are even more preferred:

| | |
|---|---|
| 0.5-10 % | active pharmaceutical ingredient, |
| 50-75 % | one or more, preferably one diluent, |
| 1-15 % | one or more binders, |
| 1-15 % | one or more disintegrants, |

wherein the percentages are by weight of the total composition.

Another pharmaceutical composition according to the first embodiment comprises preferably

| | |
|---|---|
| 0.5-20 % | active pharmaceutical ingredient, |
| 40-88 % | one or more, preferably one diluent, |
| 0.5-20 % | one or more binders, |
| 0.5-20 % | one or more disintegrants, and |
| 0.1-4 % | one or more lubricants, |

wherein the percentages are by weight of the total composition.

The following ranges are even more preferred:

| | |
|---|---|
| 0.5-10 % | active pharmaceutical ingredient, |
| 50-75 % | one or more, preferably one diluent, |
| 1-15 % | one or more binders, |
| 1-15 % | one or more disintegrants, and |
| 0.5-3 % | one or more lubricant, |

wherein the percentages are by weight of the total composition.

In the above pharmaceutical compositions the preferred diluent is mannitol. The preferred binder is copovidone. The preferred disintegrant is corn starch. A preferred lubricant is magnesium stearate. In case the pharmaceutical composition comprises a second diluent, pregelatinized starch would be preferred. It has additional binder properties.

A pharmaceutical dosage form, for example a tablet or capsule, prepared with a pharmaceutical composition according to the first embodiment contains linagliptin as the active ingredient preferably in a therapeutically effective amount. A preferred dosage range is from 0.1 to 100 mg, more preferably from 0.5 to 20 mg, even more preferably from 1 to 10 mg. Preferred dosages are for example 0.5 mg, 1 mg, 2.5 mg, 5 mg and 10 mg.

According to a second embodiment of the present invention the pharmaceutical composition comprises two active pharmaceutical ingredients which are linagliptin and a glucopyranosyl-substituted benzene derivative of the formula (I) as defined hereinbefore and hereinafter, in particular linagliptin and the compound (I.3).

Surprisingly it could be observed that a glucopyranosyl-substituted benzene derivative of the formula (I), in particular the compound (I.3), although having a glucopyranosyl-moiety with free hydroxyl-groups, is compatible with linagliptin, i.e. linagliptin combined with the glucopyranosyl-substituted benzene derivative does not show or shows only marginal degradation.

A preferred pharmaceutical composition according to the second embodiment comprises linagliptin and the compound (I.3) as the two active pharmaceutical ingredients. Preferably the pharmaceutical composition or dosage form

comprises linagliptin and the compound (I.3) wherein at least 50 % by weight of the compound (I.3) is in the form of its crystalline form (I.3X) as defined hereinbefore. More preferably in said pharmaceutical composition or dosage form at least 80 % by weight, even more preferably at least 90 % by weight of the compound (I.3) is in the form of its crystalline form (I.3X) as defined hereinbefore. Preferably the pharmaceutical composition or dosage form comprises linagliptin in one or more of the crystalline forms, in particular the polymorphs A and B, as described WO 2007/128721, which hereby is incorporated herein in its entirety.

A preferred pharmaceutical composition according to the second embodiment of the present invention comprises one or more diluents, one or more binders and one or more disintegrants. An even more preferred pharmaceutical composition according to the second embodiment of the present invention comprises one or more diluents, one or more binders, one or more disintegrants and one or more lubricants. Preferably said composition comprises one or two diluents. Even more preferably said composition does comprise one or two diluents and one binder. Even more preferably said composition does comprise one or two diluents, one binder and one disintegrant. Even more preferably said composition comprises one or two diluents, one binder and at least two disintegrants. Even more preferably said composition comprises one or two diluents, one or two binders and at least two disintegrants.

Even more preferably said composition comprises one or two diluents, one or two binders, at least two disintegrants and one lubricant. Even more preferably said composition comprises one or two diluents, one or two binders, at least two disintegrants and one or two lubricants. Even more preferably said composition comprises one or two diluents, one or two binders, at least two disintegrants and two lubricants. Even more preferably said composition comprises one or two diluents, one or two binders, three disintegrants and two lubricants. Furthermore the composition may additionally comprise at least one glidant. Preferred diluents, binders, disintegrants, lubricants and glidants are described hereinbefore and hereinafter.

A pharmaceutical composition according to the second embodiment comprises preferably

| | |
|---|---|
| 0.5-25 % | active pharmaceutical ingredient(s), |
| 40-88 % | one or more diluents, |
| 0.5-20 % | one or more binders, |
| 0.5-20 % | one or more disintegrants, |

wherein the percentages are by weight of the total composition.

The following ranges are even more preferred:

| | |
|---|---|
| 1-20 % | active pharmaceutical ingredient(s), |
| 50-75 % | one or more diluents, |
| 1-15 % | one or more binders, |
| 1-15 % | one or more disintegrants, |

wherein the percentages are by weight of the total composition.

Additionally said pharmaceutical composition may comprise one or more lubricants in a range from 0.1-15 % by weight of the total composition.

A pharmaceutical composition according to the second embodiment comprises preferably

| | |
|---|---|
| 0.5-25 % | active pharmaceutical ingredient(s), |
| 40-88 % | one or more diluents, |
| 0.5-20 % | one or more binders, |
| 0.5-20 % | one or more disintegrants, |
| 0.1-15 % | one or more lubricants |

wherein the percentages are by weight of the total composition.

In the above pharmaceutical compositions the preferred diluent is mannitol, the preferred binder is copovidone and the preferred disintegrants are selected from corn starch and crospovidone. Preferred lubricants are selected from magnesium stearate and talc. In case the pharmaceutical composition comprises a second diluent, pregelatinized starch is preferred. Pregelatinized starch has additional binder and disintegrant properties.

Therefore preferred pharmaceutical compositions according to the second embodiment are characterized by the following composition:

| | |
|---|---|
| 1-20 % | active pharmaceutical ingredients, |
| 50-75 % | mannitol, |
| 2-4 % | copovidone, |
| 8-12 % | corn starch, |

wherein the percentages are by weight of the total composition.

Another preferred pharmaceutical compositions according to the second embodiment are characterized by the following composition:

| | |
|---|---|
| 1-20 % | active pharmaceutical ingredients, |
| 50-75 % | mannitol, |
| 0-15 % | pregelatinized starch, |
| 2-4 % | copovidone, |
| 8-12 % | corn starch, |
| 0-2 % | crospovidone, |

wherein the percentages are by weight of the total composition.

Preferably the above described pharmaceutical compositions comprise additionally a lubricant. The lubricant is preferably magnesium stearate in an amount from 0.5-2 % by weight of the total composition.

Preferably the above described pharmaceutical compositions comprise additionally at least two lubricants. The first lubricant is preferably magnesium stearate in an amount from 0.5-2 % by weight of the total composition. The second lubricant is preferably talc in an amount from 0.5-10% by weight of the total composition.

Therefore preferred pharmaceutical compositions according to the second embodiment are characterized by the following composition:

| | |
|---|---|
| 1-20 % | active pharmaceutical ingredients, |
| 50-75 % | mannitol, |
| 0-15 % | pregelatinized starch, |
| 2-4 % | copovidone, |
| 8-12 % | corn starch, |
| 0-2 % | crospovidone, |
| 0.5-2 % | magnesium stearate, |

wherein the percentages are by weight of the total composition.

Another preferred pharmaceutical compositions according to the second embodiment are characterized by the following composition:

| | |
|---|---|
| 1-20 % | active pharmaceutical ingredients, |
| 50-75 % | mannitol, |
| 0-15 % | pregelatinized starch, |
| 2-4 % | copovidone, |
| 8-12 % | corn starch, |
| 0-2 % | crospovidone, |
| 0.5-2 % | magnesium stearate, |
| 0.5-10 % | talc, |

wherein the percentages are by weight of the total composition.

The pharmaceutical composition according to the invention may additionally comprise one or more taste masking agents, for example sweeteners or flavours, and pigments.

The pharmaceutical composition according to the invention may additionally comprise one or more coatings. Preferred are non-functional coatings.

The pharmaceutical compositions according to the invention are preferably solid pharmaceutical compositions, in particular intended for oral administration. A pharmaceutical dosage form according to the present invention comprising

a pharmaceutical composition according to the present invention is preferably a solid pharmaceutical dosage form, in particular for oral administration. Examples are a capsule, tablet, for example a film-coated tablet, or a granulate.

A pharmaceutical dosage form according to the first embodiment of the invention, for example a capsule or a tablet, comprises only one active pharmaceutical ingredient which is linagliptin.

A pharmaceutical dosage form according to the second embodiment of the invention, for example a capsule or a tablet, comprises two active pharmaceutical ingredients which are linagliptin and a glucopyranosyl-substituted benzene derivative of the formula (I) as defined hereinbefore and hereinafter, in particular linagliptin and the compound (I.3). The tablet may be a one-layer tablet in which the two active pharmaceutical ingredients are present in the one layer. Alternatively the tablet may be a two-layer tablet in which one of the two active pharmaceutical ingredients is present in a first layer and the other active pharmaceutical ingredient is present in a second layer. Alternatively the formulation may be a film-coated tablet in which one of the two active pharmaceutical ingredients is present in the core tablet and the other active pharmaceutical ingredient is present in the film-coating layer.

Alternatively the tablet may be a three-layer tablet in which the two layers containing only one active pharmaceutical ingredient each are separated by a third layer which does not contain any active pharmaceutical ingredient. Alternatively the tablet may be a press-coated tablet, i.e. a tablet in which the one active pharmaceutical ingredient is contained in small tablets, for example with a diameter of 2-6 mm, and the other active pharmaceutical ingredient is contained in a second granulation or blend and compressed together with one small tablet to one large press-coated tablet. All types of the hereinbeforementioned tablets may be without a coating or may have one or more coatings, in particular film-coatings. Preferred are non-functional coatings.

It will be appreciated that the amount of the one or more active pharmaceutical ingredients according to this invention to be administered to the patient and required for use in treatment or prophylaxis according to the present invention will vary with the route of administration, the nature and severity of the condition for which treatment or prophylaxis is required, the age, weight and condition of the patient, concomitant medication and will be ultimately at the discretion of the attendant physician. In general, however, a preferred amount is such that by the administration of the pharmaceutical dosage form the glycemic control in the patient to be treated is improved.

In the following preferred ranges of the amount of linagliptin and the glucopyranosyl-substituted benzene derivative to be employed in the pharmaceutical dosage form according to this invention are described. These ranges refer to the amounts to be administered per day with respect to an adult patient, in particular to a human being, for example of approximately 70 kg body weight, and can be adapted accordingly with regard to an administration 2, 3, 4 or more times daily and with regard to other routes of administration and with regard to the age of the patient. The ranges of the dosage and amounts are calculated for the individual active moiety.

A preferred pharmaceutical dosage form according to the second embodiment contains linagliptin in a therapeutically effective amount and the glucopyranosyl-substituted benzene derivative (in particular the compound (I.3)) in a therapeutically effective amount. A preferred amount of linagliptin is in a range from 0.1 to 30 mg, preferably from 0.5 to 20 mg, even more preferably from 1 to 10 mg, most preferably 2 to 5 mg. Preferred dosages are for example 0.5 mg, 1 mg, 2.5 mg, 5 mg and 10 mg. A preferred amount of the glucopyranosyl-substituted benzene derivative (in particular the compound (I.3)) is in a range from 0.5 to 100 mg, preferably from 0.5 to 50 mg, even more preferably from 1 to 25 mg, even more preferably 5 to 25 mg, most preferably 10 to 25 mg. Preferred dosages of the glucopyranosyl-substituted benzene derivative are for example 1 mg, 2 mg, 2.5 mg, 5 mg, 7.5 mg, 10 mg, 12.5 mg, 15 mg, 20 mg, 25 mg and 50 mg. A pharmaceutical dosage form according to the second embodiment contains for example a dosage combination selected from the embodiments as depicted in the following table:

| Embodiment | Amount of linagliptin | Amount of the glucopyranosyl-substituted benzene derivative, in particular of the compound (I.3) |
|---|---|---|
| E2.1 | 2 mg to 5 mg | 1 mg to 25 mg |
| E2.2 | 2 mg to 5 mg | 5 mg to 25 mg |
| E2.3 | 2 mg to 5 mg | 10 mg to 25 mg |
| E2.4 | 5 mg | 5 mg to 25 mg |
| E2.5 | 2 mg to 5 mg | 5 mg |
| E2.6 | 2 mg to 5 mg | 7.5 mg |
| E2.7 | 2 mg to 5 mg | 10 mg |
| E2.8 | 2 mg to 5 mg | 12.5 mg |
| E2.9 | 2 mg to 5 mg | 15 mg |

(continued)

| Embodiment | Amount of linagliptin | Amount of the glucopyranosyl-substituted benzene derivative, in particular of the compound (I.3) |
|---|---|---|
| E2.10 | 2 mg to 5 mg | 20 mg |
| E2.11 | 2 mg to 5 mg | 25 mg |
| E2.12 | 5 mg | 2.5 mg |
| E2.13 | 5 mg | 5 mg |
| E2.14 | 5 mg | 7.5 mg |
| E2.15 | 5 mg | 10 mg |
| E2.16 | 5 mg | 12.5 mg |
| E2.17 | 5 mg | 15 mg |
| E2.18 | 5 mg | 20 mg |
| E2.19 | 5 mg | 25 mg |
| E2.20 | 5 mg | 30 mg |
| E2.21 | 5 mg | 50 mg |

A tablet according to the invention may be film-coated. Typically a film coat represents 2-5% by weight of the total composition and comprises preferably a film-forming agent, a plasticizer, a glidant and optionally one or more pigments. An exemplary coat composition may comprise hydroxypropylmethylcellulose (HPMC), polyethylene glycol (PEG), talc, titanium dioxide and optionally iron oxide, including iron oxide red and/or yellow. An exemplary coat composition may comprise hydroxypropylmethylcellulose (HPMC), polyethylene glycol (PEG), talc, titanium dioxide, mannitol and optionally iron oxide, including iron oxide red and/or yellow.

The pharmaceutical dosage form according to the invention preferably has dissolution properties such that after 45 minutes for each of the one or two pharmaceutical active ingredients at least 75 %, even more preferably at least 90 % by weight of the respective pharmaceutical active ingredients is dissolved. In a more preferred embodiment after 30 minutes for each of the one or two pharmaceutical active ingredients at least 75 %, even more preferably at least 90 % by weight of the respective pharmaceutical active ingredients is dissolved. In a most preferred embodiment after 15 minutes for each of the one or two pharmaceutical active ingredients at least 75 %, even more preferably at least 90 % by weight of the respective pharmaceutical active ingredients is dissolved. The dissolution properties can be determined in a standard dissolution test, for example as described in pharmacopoeias, such as the USP31-NF26 S2, chapter 711 (dissolution). A preferred test is described in the experimental section.

The pharmaceutical dosage form according to the invention preferably has disintegration properties such that within 40 minutes, more preferably within 30 minutes, even more preferably within 20 minutes, most preferably within 15 minutes the pharmaceutical dosage form is disintegrated. The disintegration properties can be determined in a standard disintegration test, for example as described in pharmacopoeias, such as the USP31-NF26 S2, chapter 701 (disintegration). A preferred test is described in the experimental section.

The pharmaceutical dosage form according to the invention preferably has a high content uniformity, preferably within a range from 85 to 115 %, more preferably from 90 to 110 %, even more preferably from 95 to 105 % by weight with regard to the each of the one or two active pharmaceutical ingredients. The content uniformity can be determined in a standard test using for example randomly 30 selected pharmaceutical dosage forms, for example as described in pharmacopoeias such as the USP31-NF26 S2, chapter 905 (uniformity of dosage units).

A dosage form according to this invention, such as a tablet, capsule or film-coated tablet, may be prepared by methods well-known to the one skilled in the art.

Preferred methods of manufacturing a tablet are compression of the pharmaceutical composition in the form of a powder, i.e. direct compression, or compression of the pharmaceutical composition in the form of granules, and if needed with additional excipients.

Granules of the pharmaceutical composition according to the invention may be prepared by methods well-known to the one skilled in the art. Preferred methods for the granulation of the one or more active ingredients together with the excipients include wet granulation, for example high shear wet granulation or fluidized bed wet granulation, and dry granulation, also called roller compaction.

In a preferred wet granulation process the granulation liquid is just the solvent or mixture of solvents or a

preparation of one or more binders in a solvent or mixture of solvents. Suitable binders are described hereinbefore. An example is copovidone. Suitable solvents are for example water, ethanol, methanol, isopropanol, acetone, preferably purified water, including mixtures thereof. The solvent is a volatile component, which does not remain in the final product. The one or more active ingredients and the other excipients, in particular the one or more diluents, optionally the one or more binders and optionally the one or more disintegrants, usually with exception of the lubricant, are premixed and granulated with the granulation liquid, for example using a high shear granulator. The wet granulation step is usually followed by one or more drying and sieving steps. Optional a wet sieving step is inserted, followed by drying and dry sieving of the granules. For example a fluid bed dryer can then be used for drying.

The process for the preparation according to this invention is preferably characterized by a granulation process wherein the first and the second active pharmaceutical ingredient together with one or more diluents, one or more binders and one or more disintegrants are granulated.

The process for the preparation according to this invention is preferably characterized by a at least two granulation processes wherein in one granulation process the first active pharmaceutical ingredient together with one or more diluents, one or more binders and one or more disintegrants is granulated and in another granulation process the second active pharmaceutical ingredient together with one or more diluents, one or more binders and one or more disintegrants is granulated.

Preferably in the above processes the granulate obtained by the one or more granulation processes is optionally blended with one or more additional disintegrant and is blended with one or more lubricants.

The dried granules are sieved through an appropriate sieve. After addition of the other excipients, in particular one or more disintegrants and the glidant and optionally the lubricant talc, with exception of the lubricant, in particular magnesium stearate, the mixture is blended in a suitable blender, for example a free fall blender, followed by addition of the one or more lubricants, for example magnesium stearate, and final blending in the blender.

Thus an exemplary wet granulation process for the preparation of granules comprising the pharmaceutical composition according to the present invention comprises

a. optionally dissolving the one or more binders in a solvent or mixture of solvents such as purified water at ambient temperature to produce a granulation liquid;
b. blending the one or more active pharmaceutical ingredients, the one or more diluents, optionally the one or more binders and optionally the one or more disintegrants in a suitable mixer, to produce a pre-mix;
c. moistening the pre-mix with the granulation liquid and subsequently granulating the moistened pre-mix for example in a high shear mixer;
d. optionally sieving the granulated pre-mix through a sieve with a mesh size of at least 1.0 mm and preferably 3 mm;
e. drying the granulate at about 40-75°C and preferably 55-65°C inlet air temperature for example in a fluid bed dryer until the desired loss on drying value in the range of 1-5 % is obtained;
f. delumping the dried granulate for example by sieving through a sieve with a mesh size of 0.6 mm-1.6 mm, preferably 1.0 mm; and
g. adding preferably sieved lubricant(s) to the granulate for final blending for example in a cube mixer.

In an alternative process part of the excipients such as part of the one or more disintegrants, for example corn starch, or an additional disintegrant, for example crospovidone, and/or the one or more diluents, for example pregelatinized starch, can be added extragranularly prior to final blending of step g.

In another alternative version of the process the granulate produced in steps a to e is produced in a one pot high shear granulation process and subsequent drying in a one pot granulator. Therefore one aspect of the present invention relates to granules comprising the pharmaceutical composition of this invention.

An exemplary dry granulation process for the preparation of granules comprising the pharmaceutical composition according to the present invention comprises

(1) mixing the one or two active pharmaceutical ingredients with either all or a portion of the excipients in a mixer;
(2) compaction of the mixture of step (1) on a suitable roller compactor;
(3) reducing the ribbons obtained during step (2) to small granules by suitable milling or sieving steps;
(4) optionally mixing the granules of step (3) with the remaining excipients in a mixer to obtain the final mixture;
(5) tabletting the granules of step (3) or the final mixture of step (4) by compressing it on a suitable tablet press to produce the tablet cores;
(6) optionally film-coating of the tablet cores of step (5) with a non-functional coat.

Granules according to the first embodiment of this invention comprise only one active pharmaceutical ingredient (drug) which is linagliptin.

Granules according to the second embodiment of this invention comprise two active pharmaceutical ingredients

which are linagliptin and a glucopyranosyl-substituted benzene derivative of the formula (I) as defined hereinbefore and hereinafter, in particular linagliptin and the compound (I.3).

A preferred size of the granules is in the range from 25 to 800 μm, even more preferably from 40 μm to 500 μm. Preferably the size is measured via sieve analysis, for example with a sonic sifter. Preferably at least 80 %, more preferably at least 90 %, most preferably at least 95 % by weight of the granules is in the given range.

For the preparation of capsules the granules or the final blend for example as described above in steps (f.) and (g.) are further filled into capsules.

For the preparation of capsules according to the second embodiment of the invention granules according to the second embodiment of the invention, i.e. granules comprising the two active pharmaceutical ingredients, may be used. Alternatively granules according to the first embodiment of the invention, i.e. granules comprising linagliptin as the one active pharmaceutical ingredient, and granules comprising glucopyranosyl-substituted benzene derivative of the formula (I) as defined hereinbefore and hereinafter, in particular the compound (I.3), may be used.

For the preparation of tablets or tablet cores the granules or the final blend, for example of the above step (g.) is further compressed into tablets of the target tablet core weight with appropriate size and crushing strength, using an appropriate tablet press. The final blend comprises granules according to the invention and one or more lubricants and optionally one or more disintegrants and the optional one or more glidants. Such an additional disintegrant is crospovidone for example.

For the preparation of one-layer tablets according to the second embodiment of the invention granules according to the second embodiment of the invention, i.e. granules comprising the two active pharmaceutical ingredients, may be used. Alternatively granules according to the first embodiment of the invention, i.e. granules comprising linagliptin as the one active pharmaceutical ingredient, and granules comprising glucopyranosyl-substituted benzene derivative of the formula (I) as defined hereinbefore and hereinafter, in particular the compound (I.3), may be used.

For the preparation of two-layer tablets according to the second embodiment of the invention granules according to the first embodiment of the invention, i.e. granules comprising linagliptin as the one active pharmaceutical ingredient, may be used in a first layer and granules comprising glucopyranosyl-substituted benzene derivative of the formula (I) as defined hereinbefore and hereinafter, in particular the compound (I.3), may be used in the second layer.

A tablet, for example a one-layer tablet, according to the second embodiment of the invention comprises preferably

| | |
|---|---|
| 0.5-25 % | active pharmaceutical ingredient(s), |
| 40-88 % | one or more diluents, |
| 0.5-20 % | one or more binders, |
| 0.5-20 % | one or more disintegrants, |
| 0.1-15 % | one or more lubricants, |

wherein the percentages are by weight of the total composition.

The following ranges are even more preferred:

| | |
|---|---|
| 0.5-20 % | active pharmaceutical ingredient(s), |
| 50-75 % | one or more diluents, |
| 1-15% | one or more binders, |
| 1-15% | one or more disintegrants, |
| 0.5-10 % | one or more lubricants, |

wherein the percentages are by weight of the total composition.

Furthermore the following excipients and ranges are preferred:

| | |
|---|---|
| 0.5-20 % | active pharmaceutical ingredients, |
| 50-75 % | mannitol, (e.g. Pearlitol 50C, Roquette) |
| 0-15 % | pregelatinized starch (e.g. Maize starch 1500 INT (Colorcon)), |
| 2-4 % | copovidone (e.g. Polyvidone VA 64 INT (BASF)), |
| 8-12 % | corn starch (e.g. Maize starch undried (Roquette)), |
| 0.5-2 % | magnesium stearate (e.g. HyQual, (Mallinckrodt)), |

wherein the percentages are by weight of the total composition. An additional disintegrant, for example crospovidone,

in an amount from 0 to 2 % by weight of the total composition, may be used, in particular in cases where a higher tablet weight is achieved, such as in one-layer tablets which are made of two kinds of granules (one for each of the active ingredients) or in two-layer tablets as described above.

Furthermore the following excipients and ranges are more preferred:

| | |
|---|---|
| 0.5-20 % | active pharmaceutical ingredients, |
| 50-75 % | mannitol, (e.g. Pearlitol 50C, Roquette) |
| 0-15 % | pregelatinized starch (e.g. Maize starch 1500 INT (Colorcon)), |
| 2-4 % | copovidone (e.g. Polyvidone VA 64 INT (BASF)), |
| 8-12 % | corn starch (e.g. Maize starch undried (Roquette)), |
| 0-2 % | crospovidone (Kollidon™ CL-SF, (BASF)) |
| 0.5-2 % | magnesium stearate (e.g. HyQual, (Mallinckrodt)), |
| 0-10 % | talc (Talc, (Luzenac)) |

wherein the percentages are by weight of the total composition.

To reduce the required amount of lubricant in the tablets it is an option to use an external lubrication system.

For the preparation of film-coated tablets a coating suspension is prepared and the compressed tablet cores are coated with the coating suspension to a weight gain of about 2-5 %, preferably about 3%, using a standard film coater. The film-coating solvent is a volatile component, which does not remain in the final product. In an alternative embodiment the film-coat may comprise one of the two active pharmaceutical ingredients.

Alternatively tablets according to the invention may be prepared by direct compression. A suitable direct compression process comprises the following steps:

(1) Premixing the one or two active ingredients and the main portion of the excipients in a mixer to obtain a pre-mixture;
(2) optionally dry screening the pre-mixture through a screen in order to segregate cohesive particles and to improve content uniformity;
(3) mixing the pre-mixture of step (1) or (2) in a mixer, optionally by adding remaining excipients to the mixture and continuing mixing;
(4) tabletting the final mixture of step (3) by compressing it on a suitable tablet press to produce the tablet cores;
(5) optionally film-coating of the tablet cores of step (4) with a non-functional coat.

The pharmaceutical compositions and dosage forms, in particular tablets or capsules, according to this invention may be packaged using known packaging materials, such as PVC-blisters, PVDC-blisters, PVC/PVDC-blisters or a moisture-proof packaging material such as aluminium foil blister packs, alu/alu blister, transparent or opaque polymer blister with pouch, polypropylene tubes, glass bottles, PP bottles and HDPE bottles optionally containing a child-resistant feature (for example with a press-and-twist closure) or may be tamper evident. The primary packaging material may comprise a desiccant such as molecular sieve or silica gel to improve chemical stability of the active pharmaceutical ingredient(s). Opaque packaging such as colored blister materials, tubes, brown glass bottles or the like can be used to prolong shelflife of the active pharmaceutical ingredient(s) by reduction of photodegradation. An article for distribution may comprise the pharmaceutical composition or dosage form packaged in a packaging material as described hereinbefore and a label or package insert, which refer to instructions customarily included in commercial packages of therapeutic products, that may contain information about the indications, usage, dosage, administration, contraindications and/or warnings concerning the use of such therapeutic products. In one embodiment, the label or package insert indicates that the composition can be used for any of the purposes described herein.

The pharmaceutical compositions and pharmaceutical dosage forms according to this invention show advantageous effects in the treatment and prevention of those diseases and conditions as described hereinbefore compared with antidiabetic monotherapies. Advantageous effects may be seen for example with respect to efficacy, dosage strength, dosage frequency, pharmacodynamic properties, pharmacokinetic properties, fewer adverse effects, convenience, compliance, etc..

A pharmaceutical composition and pharmaceutical dosage form according to this invention significantly improves the glycemic control, in particular in patients as described hereinafter, compared with a monotherapy using either a SGLT2 inhibitor or a DPP IV inhibitor alone or a monotherapy of metformin. The improved glycemic control is determined as an increased lowering of blood glucose and an increased reduction of HbA1c. With monotherapy in a patient, in particular in patients as described hereinafter, the glycemic control can usually not be further improved significantly by an administration of the drug above a certain highest dose. In addition, a long term treatment using a highest dose may be unwanted in view of potential side effects. Therefore, a satisfying glycemic control cannot be achieved in all patients

via a monotherapy using either the SLGT2 inhibitor or the DPP IV inhibitor alone or another antidiabetic drug, such as metformin. In such patients a progression of the diabetes mellitus may continue and complications associated with diabetes mellitus may occur, such as macrovascular complications. The pharmaceutical composition and pharmaceutical dosage form as well as the methods according to the present invention allow a reduction of the HbA1c value to a desired target range, for example < 7 % and preferably < 6.5 %, for a higher number of patients and for a longer time of therapeutic treatment compared with an antidiabetic monotherapy.

The pharmaceutical composition and the pharmaceutical dosage form according to the present invention allow a well tolerable therapy to the patient and an improvement of the patients compliance.

A monotherapy using a DPP IV inhibitor is not independent from the insulin secretory capacity or the insulin sensitivity of a patient. On the other hand, a treatment with the administration of a SGLT2 inhibitor does not depend on the insulin secretory capacity or the insulin sensitivity of the patient. Therefore, any patient independent of the prevailing insulin levels or insulin resistance and/or hyperinsulinemia may benefit from a therapy using a pharmaceutical composition and a pharmaceutical dosage combination according to this invention. Independent of their prevailing insulin levels or their insulin resistance or hyperinsulinemia these patients can still be treated with a pharmaceutical composition and a pharmaceutical dosage because of the combined or alternate administration of the SGLT2 inhibitor.

Linagliptin according to the present invention is able - via the increases in active GLP-1 levels - to reduce the glucagon secretion in a patient. This will therefore limit the hepatic glucose production. Furthermore, the elevated active GLP-1 levels produced by linagliptin will have beneficial effects on beta-cell regeneration and neogenesis. All these features render a a pharmaceutical composition and a pharmaceutical dosage quite useful and therapeutically relevant.

When this invention refers to patients requiring treatment or prevention, it relates primarily to treatment and prevention in humans, but the pharmaceutical composition may also be used accordingly in veterinary medicine in mammals. In the scope of this invention adult patients are preferably humans of the age of 18 years or older. Also in the scope of this invention, patients are adolescent humans, i.e. humans of age 10 to 17 years, preferably of age 13 to 17 years. It is assumed that in a adolescent population the administration of the pharmaceutical composition according to the invention a very good HbA1c lowering and a very good lowering of the fasting plasma glucose can be seen. In addition it is assumed that in an adolescent population, in particular in overweight and/or obese patients, a pronounced weight loss can be observed.

As described hereinbefore by the administration of a pharmaceutical composition and a pharmaceutical dosage and in particular in view of the high SGLT2 inhibitory activity of the glucopyranosyl-substituted benzene derivative therein, excessive blood glucose is excreted through the urine of the patient, so that no gain in weight or even a reduction in body weight may result. Therefore, a treatment or prophylaxis according to this invention is advantageously suitable in those patients in need of such treatment or prophylaxis who are diagnosed of one or more of the conditions selected from the group consisting of overweight and obesity, in particular class I obesity, class II obesity, class III obesity, visceral obesity and abdominal obesity. In addition a treatment or prophylaxis according to this invention is advantageously suitable in those patients in which a weight increase is contraindicated.

The pharmaceutical composition and pharmaceutical dosage form according to this invention exhibit a very good efficacy with regard to glycemic control, in particular in view of a reduction of fasting plasma glucose, postprandial plasma glucose and/or glycosylated hemoglobin (HbA1c). By administering a pharmaceutical composition or a pharmaceutical dosage form according to this invention, a reduction of HbA1c equal to or greater than preferably 1.0 %, more preferably equal to or greater than 2.0 %, even more preferably equal to or greater than 3.0 % can be achieved and the reduction is particularly in the range from 1.0 % to 3.0 %.

Furthermore, the method and/or use according to this invention is advantageously applicable in those patients who show one, two or more of the following conditions:

(a) a fasting blood glucose or serum glucose concentration greater than 100 mg/dL, in particular greater than 125 mg/dL;
(b) a postprandial plasma glucose equal to or greater than 140 mg/dL;
(c) an HbA1c value equal to or greater than 6.5 %, in particular equal to or greater than 7.0 %, especially equal to or greater than 7.5 %, even more particularly equal to or greater than 8.0 %.

The present invention also discloses the use of the pharmaceutical composition or the pharmaceutical dosage form for improving glycemic control in patients having type 2 diabetes or showing first signs of pre-diabetes. Thus, the invention also includes diabetes prevention. If therefore a pharmaceutical composition or pharmaceutical dosage form according to this invention is used to improve the glycemic control as soon as one of the above-mentioned signs of pre-diabetes is present, the onset of manifest type 2 diabetes mellitus can be delayed or prevented.

Furthermore, the pharmaceutical composition and the pharmaceutical dosage form according to this invention is particularly suitable in the treatment of patients with insulin dependency, i.e. in patients who are treated or otherwise would be treated or need treatment with an insulin or a derivative of insulin or a substitute of insulin or a formulation

comprising an insulin or a derivative or substitute thereof. These patients include patients with diabetes type 2 and patients with diabetes type 1.

Therefore, according to a preferred embodiment of the present invention, there is provided a method for improving glycemic control and/or for reducing of fasting plasma glucose, of postprandial plasma glucose and/or of glycosylated hemoglobin HbA1c in a patient in need thereof who is diagnosed with impaired glucose tolerance (IGT), impaired fasting blood glucose (IFG) with insulin resistance, with metabolic syndrome and/or with type 2 or type 1 diabetes mellitus characterized in that a pharmaceutical composition or a pharmaceutical dosage form as defined hereinbefore and hereinafter are administered to the patient.

According to another preferred embodiment of the present invention, there is provided a method for improving glycemic control in patients, in particular in adult patients, with type 2 diabetes mellitus as an adjunct to diet and exercise.

It can be found that by using a pharmaceutical composition or a pharmaceutical dosage form according to this invention, an improvement of the glycemic control can be achieved even in those patients who have insufficient glycemic control in particular despite treatment with an antidiabetic drug, for example despite maximal recommended or tolerated dose of oral monotherapy with metformin, a SGLT2 inhibitor or a DPPIV inhibitor. A maximal recommended dose with regard to metformin is for example 2000 mg per day or 850 mg three times a day or any equivalent thereof. A maximal recommended dose with regard to a SGLT2 inhibitor according to this invention, in particular with regard to the compound (I.3), is for example 100 mg, preferably 50 mg or even 25 mg once per day or any equivalent thereof. A maximal recommended dose with regard to linagliptin is for example 10 mg, preferably 5 mg once daily or any equivalent thereof.

Therefore, the method and/or use according to this invention is advantageously applicable in those patients who show one, two or more of the following conditions:

(a) insufficient glycemic control with diet and exercise alone;
(b) insufficient glycemic control despite oral monotherapy with metformin, in particular despite oral monotherapy at a maximal recommended or tolerated dose of metformin;
(c) insufficient glycemic control despite oral monotherapy with another antidiabetic agent, in particular despite oral monotherapy at a maximal recommended or tolerated dose of the other antidiabetic agent;
(d) insufficient glycemic control despite oral monotherapy with the SGLT2 inhibitor, in particular despite oral monotherapy at a maximal recommended or tolerated dose of the SGLT2 inhibitor;
(e) insufficient glycemic control despite oral monotherapy with the DPPIV inhibitor, in particular despite oral monotherapy at a maximal recommended or tolerated dose of the DPPIV inhibitor.

The lowering of the blood glucose level by the administration of a glucopyranosyl-substituted benzene derivative according to this invention is insulin-independent. Therefore, a pharmaceutical composition according to this invention is particularly suitable in the treatment of patients who are diagnosed having one or more of the following conditions

- insulin resistance,
- hyperinsulinemia,
- pre-diabetes,
- type 2 diabetes mellitus, particular having a late stage type 2 diabetes mellitus,
- type 1 diabetes mellitus.

Furthermore, a pharmaceutical composition and a pharmaceutical dosage form according to this invention is particularly suitable in the treatment of patients who are diagnosed having one or more of the following conditions

(a) obesity (including class I, II and/or III obesity), visceral obesity and/or abdominal obesity,
(b) triglyceride blood level ≥ 150 mg/dL,
(c) HDL-cholesterol blood level < 40 mg/dL in female patients and < 50 mg/dL in male patients,
(d) a systolic blood pressure ≥ 130 mm Hg and a diastolic blood pressure ≥ 85 mm Hg,
(e) a fasting blood glucose level ≥ 100 mg/dL.

It is assumed that patients diagnosed with impaired glucose tolerance (IGT), impaired fasting blood glucose (IFG), with insulin resistance and/or with metabolic syndrome suffer from an increased risk of developing a cardiovascular disease, such as for example myocardial infarction, coronary heart disease, heart insufficiency, thromboembolic events. A glycemic control according to this invention may result in a reduction of the cardiovascular risks.

A pharmaceutical composition and a pharmaceutical dosage form according to this invention exhibits a good safety profile. Therefore, a treatment or prophylaxis according to this invention is advantageously possible in those patients for which the mono-therapy with another antidiabetic drug, such as for example metformin, is contraindicated and/or who have an intolerance against such drugs at therapeutic doses. In particular, a treatment or prophylaxis according to this invention may be advantageously possible in those patients showing or having an increased risk for one or more of the following disorders: renal insufficiency or diseases, cardiac diseases, cardiac failure, hepatic diseases, pulmonal diseases, catabolytic states and/or danger of lactate acidosis, or female patients being pregnant or during lactation.

Furthermore, it can be found that the administration of a pharmaceutical composition or a pharmaceutical dosage form according to this invention results in no risk or in a low risk of hypoglycemia. Therefore, a treatment or prophylaxis according to this invention is also advantageously possible in those patients showing or having an increased risk for hypoglycemia.

A pharmaceutical composition or a pharmaceutical dosage form according to this invention is particularly suitable in the long term treatment or prophylaxis of the diseases and/or conditions as described hereinbefore and hereinafter, in particular in the long term glycemic control in patients with type 2 diabetes mellitus.

The term "long term" as used hereinbefore and hereinafter indicates a treatment of or administration in a patient within a period of time longer than 12 weeks, preferably longer than 25 weeks, even more preferably longer than 1 year.

Therefore, a particularly preferred embodiment of the present invention provides a method for therapy, preferably oral therapy, for improvement, especially long term improvement, of glycemic control in patients with type 2 diabetes mellitus, especially in patients with late stage type 2 diabetes mellitus, in particular in patients additionally diagnosed of overweight, obesity (including class I, class II and/or class III obesity), visceral obesity and/or abdominal obesity.

In all hereinbefore and hereinafter described methods and uses, in particular the methods for treating, preventing, etc., the pharmaceutical composition or pharmaceutical dosage form according to this invention is administered to the patient preferably once daily.

Any of the above mentioned compositions and dosage forms within the scope of the invention may be tested by animal models known in the art as well as in clinical studies. In the following, *in vivo* experiments are described which are suitable to evaluate pharmacologically relevant properties of pharmaceutical compositions and dosage forms according to this invention:

> Pharmaceutical compositions, dosage forms and methods according to this invention can be tested in genetically hyperinsulinemic or diabetic animals like db/db mice, ob/ob mice, Zucker Fatty (fa/fa) rats or Zucker Diabetic Fatty (ZDF) rats. In addition, they can be tested in animals with experimentally induced diabetes like HanWistar or Sprague Dawley rats pretreated with streptozotocin.

The effect on glycemic control of the pharmaceutical compositions and dosage forms according to this invention can be tested in an oral glucose tolerance test in the animal models described hereinbefore. The time course of blood glucose is followed after an oral glucose challenge in overnight fasted animals. The compositions and dosage forms according to the present invention significantly improve glucose excursion compared to each monotherapy as measured by reduction of peak glucose concentrations or reduction of glucose AUC. In addition, after multiple dosing of the active pharmaceutical ingredients alone and the pharmaceutical compositions or dosage forms in the animal models described hereinbefore, the effect on glycemic control can be determined by measuring the HbA1 c value in blood. The compositions and dosage forms according to this invention significantly reduce HbA1 c compared to each monotherapy.

The improved independence from insulin of the treatment according to this invention can be shown after single dosing in oral glucose tolerance tests in the animal models described hereinbefore. The time course of plasma insulin is followed after a glucose challenge in overnight fasted animals. The compositions and dosage forms according to the invention will exhibit lower insulin peak concentrations or insulin AUC at lower blood glucose excursion than linagliptin alone.

The increase in active GLP-1 levels by treatment according to this invention after single or multiple dosing can be determined by measuring those levels in the plasma of animal models described hereinbefore in either the fasting or postprandial state. Likewise, a reduction in glucagon levels in plasma can be measured under the same conditions. The compositions and dosage forms according to the invention will exhibit higher active GLP-1 concentrations and lower glucagon concentrations than the glucopyranosyl-substituted benzene derivative alone.

A superior effect of the compositions and dosage forms according to the present invention on beta-cell regeneration and neogenesis can be determined after multiple dosing in the animal models described hereinbefore by measuring the increase in pancreatic insulin content, or by measuring increased beta-cell mass by morphometric analysis after immunhistochemical staining of pancreatic sections, or by measuring increased glucose-stimulated insulin secretion in isolated pancreatic islets.

## Pharmacological Examples

The following examples show the beneficial effect on glycemic control of the combination according to the present invention.

Example I:

According to a first example an oral glucose tolerance test is performed in overnight fasted 9-weeks old male Zucker Diabetic Fatty (ZDF) rats (ZDF/Crl-$Lepr^{fa}$). A pre-dose blood sample is obtained by tail bleed. Blood glucose is measured with a glucometer, and the animals are randomized for blood glucose (n = 5 / group). Subsequently, the groups receive a single oral administration of either vehicle alone (0.5% aqueous hydroxyethylcellulose containing 3 mM HCl and 0.015% Polysorbat 80) or vehicle containing either the SGLT2 inhibitor or the DPPIV inhibitor or the combination of the SGLT2 inhibitor plus the DPP IV inhibitor plus. The animals receive an oral glucose load (2 g/kg) 30 min after compound administration. Blood glucose is measured in tail blood 30 min, 60 min, 90 min, 120 min, and 180 min after the glucose challenge. Glucose excursion is quantified by calculating the reactive glucose AUC. The data are presented as mean ± SEM. The two-sided unpaired Student t-test is used for statistical comparison of the control group and the active groups.

The result is shown in Figure 3. "Cpd. A" is linagliptin at a dose of 1 mg/kg. Cpd. B is the compound (I.3), i.e. 1-chloro-4-(β-D-glucopyranos-1-yl)-2-[4-((*S*)-tetrahydrofuran-3-yloxy)-benzyl]-benzene, at a dose of 3 mg/kg. Combination A + B is the combination of linagliptin and the compound (I.3) at the same doses. P-values versus control are indicated by symbols above the bars. P-values of the combination versus the monotherapies are indicated below the figure (*, p < 0.05; **, p < 0.01; ***, p < 0.001). Linagliptin reduces glucose excursion by 56%, the compound (I.3) reduces glucose excursion by 51%. The combination decreased glucose excursion in the oral glucose tolerance test by 84%, and this reduction in glucose AUC is statistically significant versus each monotherapy.

Example **II:**

According to a second example an oral glucose tolerance test is performed in overnight fasted male Sprague Dawley rats (Crl:CD(SD)) with a body weight of about 200 g. A pre-dose blood sample is obtained by tail bleed. Blood glucose is measured with a glucometer, and the animals are randomized for blood glucose (n = 5 / group). Subsequently, the groups receive a single oral administration of either vehicle alone (0.5% aqueous hydroxyethylcellulose containing 0.015% Polysorbat 80) or vehicle containing either the SGLT2 inhibitor or the DPPIV inhibitor or the third antidiabetic agent or the combination of the SGLT2 inhibitor plus the DPP IV inhibitor plus the third antidiabetic agent. Alternatively the groups receive a single oral administration of either vehicle alone or vehicle containing either the SGLT2 inhibitor or the DPPIV inhibitor plus the third antidiabetic agent or the third antidiabetic agent or the combination of the SGLT2 inhibitor plus the DPP IV inhibitor plus the third antidiabetic agent. The animals receive an oral glucose load (2 g/kg) 30 min after compound administration. Blood glucose is measured in tail blood 30 min, 60 min, 90 min, and 120 min after the glucose challenge. Glucose excursion is quantified by calculating the reactive glucose AUC. The data are presented as mean ± S.E.M. Statistical comparisons are conducted by Student's *t* test.

**Example III: Treatment of pre-diabetes**

The efficacy of a pharmaceutical composition or pharmaceutical dosage form according to the invention in the treatment of pre-diabetes characterised by pathological fasting glucose and/or impaired glucose tolerance can be tested using clinical studies. In studies over a shorter period (e.g. 2-4 weeks) the success of the treatment is examined by determining the fasting glucose values and/or the glucose values after a meal or after a loading test (oral glucose tolerance test or food tolerance test after a defined meal) after the end of the period of therapy for the study and comparing them with the values before the start of the study and/or with those of a placebo group. In addition, the fructosamine value can be determined before and after therapy and compared with the initial value and/or the placebo value. A significant drop in the fasting or non-fasting glucose levels demonstrates the efficacy of the treatment. In studies over a longer period (12 weeks or more) the success of the treatment is tested by determining the HbA1c value, by comparison with the initial value and/or with the value of the placebo group. A significant change in the HbA1 c value compared with the initial value and/or the placebo value demonstrates the efficacy of the composition or dosage form according to the invention for treating pre-diabetes.

**Example IV: Preventing manifest type 2 diabetes**

Treating patients with pathological fasting glucose and/or impaired glucose tolerance (pre-diabetes) is also in pursuit of the goal of preventing the transition to manifest type 2 diabetes. The efficacy of a treatment can be investigated in a comparative clinical study in which pre-diabetes patients are treated over a lengthy period (e.g. 1-5 years) with either a pharmaceutical composition according to this invention or with placebo or with a non-drug therapy or other medicaments. During and at the end of the therapy, by determining the fasting glucose and/or a loading test (e.g. oGTT), a check is made to determine how many patients exhibit manifest type 2 diabetes, for example a fasting glucose level of >125

mg/dl and/or a 2h value according to oGTT of >199 mg/dl. A significant reduction in the number of patients who exhibit manifest type 2 diabetes when treated with a pharmaceutical composition or dosage form according to this invention as compared to one of the other forms of treatment, demonstrates the efficacy in preventing a transition from pre-diabetes to manifest diabetes.

**Example V: Treatment of type 2 diabetes**

Treating patients with type 2 diabetes with the pharmaceutical composition or dosage form according to the invention, in addition to producing an acute improvement in the glucose metabolic situation, prevents a deterioration in the metabolic situation in the long term. This can be observed is patients are treated for a longer period, e.g. 3 months to 1 year or even 1 to 6 years, with the pharmaceutical composition or dosage form according to the invention and are compared with patients who have been treated with placebo or other antidiabetic medicaments. There is evidence of therapeutic success compared with patients treated with placebo or other antidiabetic medicaments if no or only a slight increase in the fasting glucose and/or HbA1c value is observed. Further evidence of therapeutic success is obtained if a significantly smaller percentage of the patients treated with a pharmaceutical composition or dosage form according to the invention, compared with patients who have been treated with other medicaments, undergo a deterioration in the glucose metabolic position (e.g. an increase in the HbA1c value to >6.5% or >7%) to the point where treatment with an additional oral antidiabetic medicament or with insulin or with an insulin analogue is indicated.

**Example VI: Treatment of insulin resistance**

In clinical studies running for different lengths of time (e.g. 2 weeks to 12 months) the success of the treatment is checked using a hyperinsulinaemic euglycaemic glucose clamp study. A significant rise in the glucose infusion rate at the end of the study, compared with the initial value or compared with a placebo group, or a group given a different therapy, proves the efficacy of a pharmaceutical composition or dosage form according to the invention in the treatment of insulin resistance.

**Example VII: Treatment of hyperglycaemia**

In clinical studies running for different lengths of time (e.g. 1 day to 24 months) the success of the treatment in patients with hyperglycaemia is checked by determining the fasting glucose or non-fasting glucose (e.g. after a meal or a loading test with oGTT or a defined meal). A significant fall in these glucose values during or at the end of the study, compared with the initial value or compared with a placebo group, or a group given a different therapy, proves the efficacy of a pharmaceutical composition or dosage form according to the invention in the treatment of hyperglycaemia.

**Example VIII: Prevention of micro- or macrovascular complications**

The treatment of type 2 diabetes or pre-diabetes patients with a pharmaceutical composition or dosage form according to the invention prevents or reduces or reduces the risk of developing microvascular complications (e.g. diabetic neuropathy, diabetic retinopathy, diabetic nephropathy, diabetic foot, diabetic ulcer) or macrovascular complications (e.g. myocardial infarct, acute coronary syndrome, unstable angina pectoris, stable angina pectoris, stroke, peripheral arterial occlusive disease, cardiomyopathy, heart failure, heart rhythm disorders, vascular restenosis). Type 2 diabetes or patients with pre-diabetes are treated long-term, e.g. for 1-6 years, with a pharmaceutical composition or dosage form according to the invention or a combination of active ingredients according to the invention and compared with patients who have been treated with other antidiabetic medicaments or with placebo. Evidence of the therapeutic success compared with patients who have been treated with other antidiabetic medicaments or with placebo can be found in the smaller number of single or multiple complications. In the case of macrovascular events, diabetic foot and/or diabetic ulcer, the numbers are counted by anamnesis and various test methods. In the case of diabetic retinopathy the success of the treatment is determined by computer-controlled illumination and evaluation of the background to the eye or other ophthalmic methods. In the case of diabetic neuropathy, in addition to anamnesis and clinical examination, the nerve conduction rate can be measured using a calibrated tuning fork, for example. With regard to diabetic nephropathy the following parameters may be investigated before the start, during and at the end of the study: secretion of albumin, creatinin clearance, serum creatinin values, time taken for the serum creatinin values to double, time taken until dialysis becomes necessary.

**Example IX: Treatment of Metabolic Syndrome**

The efficacy of a pharmaceutical composition or dosage form according to the invention can be tested in clinical

studies with varying run times (e.g. 12 weeks to 6 years) by determining the fasting glucose or non-fasting glucose (e.g. after a meal or a loading test with oGTT or a defined meal) or the HbA1c value. A significant fall in these glucose values or HbA1c values during or at the end of the study, compared with the initial value or compared with a placebo group, or a group given a different therapy, proves the efficacy of a pharmaceutical composition or dosage form according to this invention in the treatment of Metabolic Syndrome. Examples of this are a reduction in systolic and/or diastolic blood pressure, a lowering of the plasma triglycerides, a reduction in total or LDL cholesterol, an increase in HDL cholesterol or a reduction in weight, either compared with the starting value at the beginning of the study or in comparison with a group of patients treated with placebo or a different therapy.

## Examples of Pharmaceutical Compositions and Pharmaceutical Dosage Forms

In the following the term "API 1" denotes a glucopyranosyl-substituted benzene derivative of the formula (I), in particular the compound (I.3), preferably in its crystalline form (I3.X), and the term "API 2" denotes linagliptin.

The active pharmaceutical ingredients, i.e. linagliptin and the compound (I.3), preferably in the crystalline form (I3.X), are milled with a suitable mill like pin-mill or jet-mill in order to obtain the desired particle size distribution before manufacturing of the pharmaceutical composition or dosage form.

Examples of typical particle size distribution values X90, X50 and X10 for the preferred active pharmaceutical ingredients according to the invention are shown in the table below.

| | API 1 Batch 1 | API 1 Batch 2 | API 2 Batch 1 | API 2 Batch 2 |
|---|---|---|---|---|
| X10 | 1,8 μm | 1,7 μm | 2,1 μm | 2,0 μm |
| X50 | 18,9 μm | 12,1 μm | 13,5 μm | 17,3 μm |
| X90 | 45,3 μm | 25,9 μm | 31,8 μm | 36,8 μm |

### Example 1: One granulation, mono-layer tablet

Copovidone is dissolved in purified water at ambient temperature (about 20°C) to produce a granulation liquid. The API 2 and API 1, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

Magnesium stearate is passed through a sieve for delumping and added to the granulate. Subsequently the final blend is produced by final blending in a suitable blender for three minutes and compressed into 8 mm round tablet cores with a compression force of 15 kN.

Hydroxypropyl methylcellulose, polyethylene glycol, talc, titanium dioxide and iron oxide are suspended in purified water in a suitable mixer at ambient temperature to produce a coating suspension. The tablet cores are coated with the coating suspension to a weight gain of about 3 % to produce film-coated tablets. The following formulation variants can be obtained:

| Ingredient | mg/ / tablet | mg/ / tablet | mg/ / tablet | mg/ / tablet | mg/ tablet |
|---|---|---|---|---|---|
| API 1 | 2.5 | 5.0 | 10.0 | 25.0 | 50.0 |
| API 2 | 5.0 | 5.0 | 5.0 | 5.0 | 5.0 |
| Mannitol | 128.4 | 125.9 | 120.9 | 105.9 | 80.9 |
| Pregelatinised starch | 18.0 | 18.0 | 18.0 | 18.0 | 18.0 |
| Maize starch | 18.0 | 18.0 | 18.0 | 18.0 | 18.0 |
| Copovidone | 5.4 | 5.4 | 5.4 | 5.4 | 5.4 |
| Magnesium stearate | 2.7 | 2.7 | 2.7 | 2.7 | 2.7 |
| Film coat | 5.0 | 5.0 | 5.0 | 5.0 | 5.0 |
| Total | 185.0 | 185.0 | 185.0 | 185.0 | 185.0 |

The resulting tablets have a tablet hardness around 85 N, the friability is below 0.5%. The content uniformity

fulfills the requirement according to the USP. The disintegration time is around 7 minutes and the dissolution of both API 1 and API 2 is > 85% after 15 minutes, e.g. 97% of API 1 and 101% of API 2.

**Example 2: One granulation, mono-layer tablet**

Copovidone is dissolved in purified water at ambient temperature to produce a granulation liquid. The API 1, API 2, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

Magnesium stearate is passed through a sieve for delumping and added to the granulate. Subsequently the final blend is produced by final blending in a suitable blender for three minutes and compressed into 8 mm round tablet cores with a compression force of 17 kN.

Hydroxypropyl methylcellulose, polyethylene glycol, talc, titanium dioxide and iron oxide are suspended in purified water in a suitable mixer at ambient temperature to produce a coating suspension. The tablet cores are coated with the coating suspension to a weight gain of about 3 % to produce film-coated tablets. The following formulation variants can be obtained:

| Ingredient | mg/ tablet | mg/ tablet | mg/ tablet | mg/ tablet | mg/ tablet |
|---|---|---|---|---|---|
| API 1 | 2.5 | 5.0 | 10.0 | 25.0 | 50.0 |
| API 2 | 5.0 | 5.0 | 5.0 | 5.0 | 5.0 |
| Mannitol | 127.5 | 125.0 | 120.0 | 105.0 | 80.0 |
| Pregelatinised starch | 18.0 | 18.0 | 18.0 | 18.0 | 18.0 |
| Maize starch | 18.0 | 18.0 | 18.0 | 18.0 | 18.0 |
| Copovidone | 5.4 | 5.4 | 5.4 | 5.4 | 5.4 |
| Magnesium stearate | 3.6 | 3.6 | 3.6 | 3.6 | 3.6 |
| Film coat | 5.0 | 5.0 | 5.0 | 5.0 | 5.0 |
| Total | 185.0 | 185.0 | 185.0 | 185.0 | 185.0 |

The tablet hardness, the friability, the content uniformity, the disintegration time and the dissolution properties are determined as described hereinbefore.

**Example 3: One granulation, mono-layer tablet**

Copovidone is dissolved in purified water at ambient temperature to produce a granulation liquid. API 1, API 2, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm. Crospovidone is added to the dried granulate and mixed for 5 minutes to produce the main blend. Magnesium stearate is passed through a sieve for delumping and added to main blend. Subsequently the final blend is produced by final blending in a suitable blender for three minutes and compressed into 8 mm round tablet cores with a compression force of 16 kN.

Hydroxypropyl methylcellulose, polyethylene glycol, talc, titanium dioxide and iron oxide are suspended in purified water in a suitable mixer at ambient temperature to produce a coating suspension. The tablet cores are coated with the coating suspension to a weight gain of about 3 % to produce film-coated tablets. The following formulation variants can be obtained:

| Ingredient | mg/ tablet | mg/ tablet | mg/ tablet | mg/ tablet | mg/ tablet |
|---|---|---|---|---|---|
| API 1 | 2.5 | 5.0 | 10.0 | 25.0 | 50.0 |
| API 2 | 5.0 | 5.0 | 5.0 | 5.0 | 5.0 |
| Mannitol | 127.5 | 125.0 | 120.0 | 105.0 | 80.0 |

(continued)

| Ingredient | mg/ tablet | mg/ tablet | mg/ tablet | mg/ tablet | mg/ tablet |
|---|---|---|---|---|---|
| Pregelatinised starch | 18.0 | 18.0 | 18.0 | 18.0 | 18.0 |
| Maize starch | 18.0 | 18.0 | 18.0 | 18.0 | 18.0 |
| Crospovidone | 2.0 | 2.0 | 2.0 | 2.0 | 2.0 |
| Copovidone | 5.4 | 5.4 | 5.4 | 5.4 | 5.4 |
| Magnesium stearate | 3.6 | 3.6 | 3.6 | 3.6 | 3.6 |
| Film coat | 5.0 | 5.0 | 5.0 | 5.0 | 5.0 |
| Total | 187.0 | 187.0 | 187.0 | 187.0 | 187.0 |

The tablet hardness, the friability, the content uniformity, the disintegration time and the dissolution properties are determined as described hereinbefore.

**Example 4: Two granulations, mono-layer tablet**

Two separate granulations containing only one active pharmaceutical ingredient each are prepared. For both granulations, copovidone is dissolved in purified water at ambient temperature to produce a granulation liquid.

The API 2, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

The API 1, mannitol, pregelatinized starch, corn starch and optionally pigments like iron oxides red are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

The two granulates are combined, crospovidone is added and all components are mixed for 5 minutes in a suitable mixer to produce the main blend. Magnesium stearate is passed through a sieve for delumping and added to main blend. Subsequently the final blend is produced by final blending in a suitable blender for three minutes and compressed into 15x6 mm oval-shaped tablet cores with a compression force of 17 kN. The following formulation variants can be obtained:

| Ingredient | mg / tablet | mg / tablet | mg / tablet | mg / tablet | mg / tablet |
|---|---|---|---|---|---|
| 1 st granulation | | | | | |
| API 1 | 2.5 | 5.0 | 10.0 | 25.0 | 50.0 |
| Mannitol | 123.5 | 121.0 | 116.0 | 101.0 | 76.0 |
| Pregelatinised starch | 18.0 | 18.0 | 18.0 | 18.0 | 18.0 |
| Maize starch | 18.0 | 18.0 | 18.0 | 18.0 | 18.0 |
| Iron oxide red | 2.7 | 2.7 | 2.7 | 2.7 | 2.7 |
| Copovidone | 5.4 | 5.4 | 5.4 | 5.4 | 5.4 |
| 2nd granulation | | | | | |
| API 2 | 5.0 | 5.0 | 5.0 | 5.0 | 5.0 |
| Mannitol | 130.9 | 130.9 | 130.9 | 130.9 | 130.9 |
| Pregelatinised starch | 18.0 | 18.0 | 18.0 | 18.0 | 18.0 |
| Maize starch | 18.0 | 18.0 | 18.0 | 18.0 | 18.0 |
| Copovidone | 5.4 | 5.4 | 5.4 | 5.4 | 5.4 |
| Final Blend | | | | | |

(continued)

| Ingredient | mg / tablet | mg / tablet | mg / tablet | mg / tablet | mg / tablet |
|---|---|---|---|---|---|
| 1 st granulation | | | | | |
| Magnesium stearate | 7.2 | 7.2 | 7.2 | 7.2 | 7.2 |
| Crospovidone | 5.4 | 5.4 | 5.4 | 5.4 | 5.4 |
| Total | **360.0** | **360.0** | **360.0** | **360.0** | **360.0** |

The resulting tablets have a tablet hardness around 105 N. The content uniformity fulfills the requirement according to the USP. The friability is below 0.5%. The disintegration time is around 5 minutes and the dissolution of both APIs is > 85% after 15 minutes.

**Example 5: Two granulations, mono-layer tablet**

Two separate granulations containing only one active pharmaceutical ingredient each are prepared.

Copovidone is dissolved in purified water at ambient temperature to produce a granulation liquid. The API 2, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

The API 1, mannitol, microcrystalline cellulose, hydroxypropyl cellulose and optionally pigments like iron oxides red are blended in a suitable mixer, to produce a pre-mix. The pre-mix is moistened with purified water and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

The two granulates are combined, crospovidone is added and all components are mixed for 5 minutes in a suitable mixer to produce the main blend. Magnesium stearate is passed through a sieve for delumping and added to main blend. Subsequently the final blend is produced by final blending in a suitable blender for three minutes and compressed into 15x6 mm oval-shaped tablet cores with a compression force of 15 kN. The following formulation variants can be obtained:

| Ingredient | mg / tablet | mg / tablet | mg / tablet | mg / tablet | mg / tablet |
|---|---|---|---|---|---|
| 1 st granulation | | | | | |
| API 1 | 2.5 | 5.0 | 10.0 | 25.0 | 50.0 |
| Mannitol | 123.5 | 121.0 | 116.0 | 101.0 | 76.0 |
| Microcrystalline cellulose | 36.0 | 36.0 | 36.0 | 36.0 | 36.0 |
| Iron oxide red | 2.7 | 2.7 | 2.7 | 2.7 | 2.7 |
| Hydroxypropyl cellulose | 5.4 | 5.4 | 5.4 | 5.4 | 5.4 |
| 2nd granulation | | | | | |
| API 2 | 5.0 | 5.0 | 5.0 | 5.0 | 5.0 |
| Mannitol | 130.9 | 130.9 | 130.9 | 130.9 | 130.9 |
| Pregelatinised starch | 18.0 | 18.0 | 18.0 | 18.0 | 18.0 |
| Maize starch | 18.0 | 18.0 | 18.0 | 18.0 | 18.0 |
| Copovidone | 5.4 | 5.4 | 5.4 | 5.4 | 5.4 |
| Final Blend | | | | | |
| Magnesium stearate | 7.2 | 7.2 | 7.2 | 7.2 | 7.2 |
| Crospovidone | 5.4 | 5.4 | 5.4 | 5.4 | 5.4 |
| Total | **360.0** | **360.0** | **360.0** | **360.0** | **360.0** |

The tablet hardness, the friability, the content uniformity, the disintegration time and the dissolution properties are determined as described hereinbefore.

**Example 6: Two granulations, bi-layer tablet**

Two separate granulations containing only one active pharmaceutical ingredient each are prepared. For both granulations, copovidone is dissolved in purified water at ambient temperature to produce a granulation liquid.

The API 2, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

The API 1, mannitol, pregelatinized starch, corn starch and optionally pigments like iron oxides red are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm, crospovidone is added and the components are mixed in a suitable mixer for 5 minutes.

Magnesium stearate is passed through a sieve for delumping and added to the two granulations separately. Subsequently two final blends are produced by final blending in a suitable blender for three minutes. The final blend containing API 1 is used for the first layer and the final blend containing API 2 is used for the second layer of the bi-layer tablet. The bi-layer tablets are produced on a suitable tablet press with a first compression force of 2 kN for the first layer and a main compression force of 12 kN for producing 10 mm round tablet cores. The following formulation variants can be obtained:

| Ingredient | mg / tablet | mg / tablet | mg / tablet | mg / tablet | mg / tablet |
|---|---|---|---|---|---|
| 1 st layer | | | | | |
| API 1 | 2.5 | 5.0 | 10.0 | 25.0 | 50.0 |
| Mannitol | 123.5 | 121.0 | 116.0 | 101.0 | 76.0 |
| Pregelatinised starch | 18.0 | 18.0 | 18.0 | 18.0 | 18.0 |
| Maize starch | 18.0 | 18.0 | 18.0 | 18.0 | 18.0 |
| Iron oxide red | 2.7 | 2.7 | 2.7 | 2.7 | 2.7 |
| Copovidone | 5.4 | 5.4 | 5.4 | 5.4 | 5.4 |
| Magnesium stearate | 4.5 | 4.5 | 4.5 | 4.5 | 4.5 |
| Crospovidone | 5.4 | 5.4 | 5.4 | 5.4 | 5.4 |
| 2nd layer | | | | | |
| API 2 | 5.0 | 5.0 | 5.0 | 5.0 | 5.0 |
| Mannitol | 130.9 | 130.9 | 130.9 | 130.9 | 130.9 |
| Pregelatinised starch | 18.0 | 18.0 | 18.0 | 18.0 | 18.0 |
| Maize starch | 18.0 | 18.0 | 18.0 | 18.0 | 18.0 |
| Copovidone | 5.4 | 5.4 | 5.4 | 5.4 | 5.4 |
| Magnesium stearate | 2.7 | 2.7 | 2.7 | 2.7 | 2.7 |
| Total | **360.0** | **360.0** | **360.0** | **360.0** | **360.0** |

The resulting tablets have a tablet hardness around 120 N, the friability is below 0.5%. The content uniformity fulfills the requirement according to the USP. The disintegration time is around 6 minutes and the dissolution of both APIs is > 85% after 15 minutes.

**Example 7: Two granulations, bi-layer tablet**

Two separate granulations containing only one active pharmaceutical ingredient each are prepared.

Copovidone is dissolved in purified water at ambient temperature to produce a granulation liquid. The API 2, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

The API 1, mannitol, microcrystalline cellulose, hydroxypropyl cellulose and optionally pigments like iron oxides red are blended in a suitable mixer, to produce a pre-mix. The pre-mix is moistened with purified water and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm, crospovidone is added and the components are mixed in a suitable mixer for 5 minutes.

Magnesium stearate is passed through a sieve for delumping and added to the two granulations separately. Subsequently two final blends are produced by final blending in a suitable blender for three minutes. The final blend containing API 1 is used for the first layer and the final blend containing API 2 is used for the second layer of the bi-layer tablet. The bi-layer tablets are produced on a suitable tablet press with a first compression force of 2 kN for the first layer and a main compression force of 12 kN for producing 10 mm round tablet cores. The following formulation variants can be obtained:

| Ingredient | mg / tablet | mg / tablet | mg / tablet | mg / tablet | mg / tablet |
|---|---|---|---|---|---|
| 1 st layer | | | | | |
| API 1 | 2.5 | 5.0 | 10.0 | 25.0 | 50.0 |
| Mannitol | 123.5 | 121.0 | 116.0 | 101.0 | 76.0 |
| Microcrystalline cellulose | 36.0 | 36.0 | 36.0 | 36.0 | 36.0 |
| Iron oxide red | 2.7 | 2.7 | 2.7 | 2.7 | 2.7 |
| Hydroxypropyl cellulose | 5.4 | 5.4 | 5.4 | 5.4 | 5.4 |
| Magnesium stearate | 4.5 | 4.5 | 4.5 | 4.5 | 4.5 |
| Crospovidone | 5.4 | 5.4 | 5.4 | 5.4 | 5.4 |
| 2nd layer | | | | | |
| API 2 | 5.0 | 5.0 | 5.0 | 5.0 | 5.0 |
| Mannitol | 130.9 | 130.9 | 130.9 | 130.9 | 130.9 |
| Pregelatinised starch | 18.0 | 18.0 | 18.0 | 18.0 | 18.0 |
| Maize starch | 18.0 | 18.0 | 18.0 | 18.0 | 18.0 |
| Copovidone | 5.4 | 5.4 | 5.4 | 5.4 | 5.4 |
| Magnesium stearate | 2.7 | 2.7 | 2.7 | 2.7 | 2.7 |
| Total | **360.0** | **360.0** | **360.0** | **360.0** | **360.0** |

The tablet hardness, the friability, the content uniformity, the disintegration time and the dissolution properties are determined as described hereinbefore.

**Example 8: One granulation, mono-layer tablet**

Copovidone is dissolved in purified water at ambient temperature (about 20°C) to produce a granulation liquid. API 1, API 2, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

Crospovidone and talc are added to the dried granulate and mixed for 5 minutes to produce the main blend. Magnesium stearate is passed through a sieve for delumping and added to main blend. Subsequently the final blend is produced by final blending in a suitable blender for three minutes and compressed into 8 mm round tablet cores with a compression force of 16 kN. The combination of the two lubricants talc and magnesium stearate was discovered to be especially useful when API 1 and API 2 are combined in one granulation and subsequently in one tablet by enabling

low ejection forces and by avoiding sticking of the final blend to the tablet punches.

Hydroxypropyl methylcellulose, polyethylene glycol, talc, titanium dioxide, mannitol and iron oxide are suspended in purified water in a suitable mixer at ambient temperature to produce a coating suspension. The tablet cores are coated with the coating suspension to a weight gain of about 3 % to produce film-coated tablets. The following formulation variants can be obtained:

| Ingredient | mg/ tablet | mg/ tablet | mg/ tablet | mg/ tablet | mg/ tablet |
|---|---|---|---|---|---|
| API 1 | 2.5 | 5.0 | 10.0 | 25.0 | 50.0 |
| API 2 | 5.0 | 5.0 | 5.0 | 5.0 | 5.0 |
| Mannitol | 114.0 | 111.5 | 106.5 | 91.5 | 66.5 |
| Pregelatinised starch | 18.0 | 18.0 | 18.0 | 18.0 | 18.0 |
| Maize starch | 19.8 | 19.8 | 19.8 | 19.8 | 19.8 |
| Crospovidone | 3.6 | 3.6 | 3.6 | 3.6 | 3.6 |
| Copovidone | 5.4 | 5.4 | 5.4 | 5.4 | 5.4 |
| Talc | 9.0 | 9.0 | 9.0 | 9.0 | 9.0 |
| Magnesium stearate | 2.7 | 2.7 | 2.7 | 2.7 | 2.7 |
| Hydoxypropyl methylcellulose | 1.7500 | 1.7500 | 1.7500 | 1.7500 | 1.7500 |
| Polyethylene glycol | 0.6000 | 0.6000 | 0.6000 | 0.6000 | 0.6000 |
| Iron oxides | 0.0125 | 0.0125 | 0.0125 | 0.0125 | 0.0125 |
| Titanium dioxide | 0.7375 | 0.7375 | 0.7375 | 0.7375 | 0.7375 |
| Talc | 0.9000 | 0.9000 | 0.9000 | 0.9000 | 0.9000 |
| Mannitol | 1.0000 | 1.0000 | 1.0000 | 1.0000 | 1.0000 |
| Total | 185.0 | 185.0 | 185.0 | 185.0 | 185.0 |

The tablet hardness, the friability, the content uniformity, the disintegration time and the dissolution properties are determined as described hereinbefore.

**<u>Examples of Tests with regard to Properties of Pharmaceutical Compositions and Pharmaceutical Dosage Forms</u>**

### 1. Disintegration Test

Disintegration test was performed as described in USP31-NF26 S2, chapter 701 (disintegration).

### 2. Dissolution Test

The standard dissolution test is described in USP31-NF26 S2, chapter 711 (dissolution). The paddle method (Apparatus 2) with an agitation speed of 50 rpm was used. The dissolution media is 900 mL 0.05 M Potassium phosphate buffer pH 6.8 at a temperature of 37°C. Samples are taken after 10, 15, 20, 30 and 45 minutes. The samples are analyzed via HPLC.

A dissolution profile of tablets according to the example 4 and the example 6 wherein API 1 is the compound (I.3) and the API 2 is linagliptin are depicted in the Figure 4.

A dissolution profile of tablets according to the example 8 wherein API 1 is the compound (I.3) and the API 2 is linagliptin are depicted in the Figure 5.

### 3. Particle Size Distribution Measurement by Laser Diffraction

Particle size distribution measurement was performed for example via light scattering or laser diffraction technique. To determine the particle size the powder is fed into a laser diffraction spectrometer for example by means of a dispersing unit. The test method is described below in detail:

| | |
|---|---|
| Equipment: | Laser Diffraction Spectrometer Sympatec HELOS Particle Sizer. |
| Lens: | R31 (0.5/0.9μm - 175μm) |
| Sample Dispersing Unit: | Dry disperser RODOS/M |
| Vacuum: | Nilfisk |
| Feeder: | ASPIROS |
| Feed Velocity: | 60.00 mm/s |
| Primary pressure: | 2.00 bar |
| Injector depression: | maximize (mbar)2 |
| Reference Measurement: | 10 seconds |
| Cycle Time: | 100 msec |
| Trigger Conditions: | Start 0.0 seconds after optical concentration ≥ 1% valid always |
| Stop after 5.0 seconds optical concentration ≤ 1% or after 30 seconds real time | |
| Optical Concentration: | Approximately range 3 - 12 % |
| Evaluation: | HRLD |
| Sample Size: | Approximately 100 mg |
| Number of measurements: | 2 (duplicate) |

The instrument is set up according to the manufacturer's recommendation and using the manufacturer provided software. The sample container is thoroughly mixed and tumbled prior to removing a portion of the sample to ensure that a representative sample is tested.
Duplicate samples are prepared by using a spatula to transfer approximately 100 mg of a sample into the ASPIROS glass vials and cap the vials. The capped vials are placed into the feeder.

**4. Tablet hardness and friability**

Tablet hardness and friability test was performed as described in USP31-NF26 S2, chapter 1217 (tablet breaking force).

**Claims**

**1.** A pharmaceutical composition comprising linagliptin as a first active pharmaceutical ingredient and a glucopyranosyl-substituted benzene derivative of the formula (I)

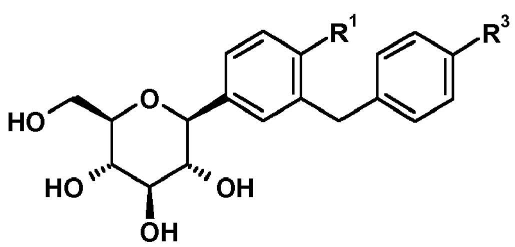

I

wherein $R^1$ denotes chloro or methyl; and $R^3$ denotes (*S*)-tetrahydrofuran-3-yloxy as a second active pharmaceutical ingredient and one or more excipients.

**2.** The pharmaceutical composition according to the claim 1 wherein the first active ingredient has a particle size distribution of X90 < 200 μm.

**3.** The pharmaceutical composition according to the claim 1 or 2 wherein the second active ingredient has a particle size distribution of 1 μm < X90 < 200 μm.

**4.** The pharmaceutical composition according to claim 1, 2 or 3 wherein the one or more excipients comprise one or more diluents and one or more binders.

**5.** The pharmaceutical composition according to claim 1, 2 or 3 wherein the one or more excipients comprise one or more diluents, one or more binders and one or more disintegrants.

**6.** The pharmaceutical composition according to one or more of the previous claims comprising

| | |
|---|---|
| 0.5-25 % | of the two active ingredients, |
| 40-88 % | of the one or more diluents, |
| 0.5-20 % | of the one or more binders, and |
| 0.5-20 % | of the one or more disintegrants, |

wherein the percentages are by weight of the total composition.

**7.** A pharmaceutical dosage form comprising a pharmaceutical composition according to one or more of the claims 1 to 6 **characterized in that** it is a solid pharmaceutical dosage form, in particular a capsule or a tablet.

**8.** The pharmaceutical dosage form according to the claim 7 comprising linagliptin as a first active pharmaceutical ingredient in an amount from 0.1 to 30 mg and a glucopyranosyl-substituted benzene derivative of the formula (I) as defined in claim 1 as a second active pharmaceutical ingredient in an amount from 0.5 to 100 mg.

**9.** The pharmaceutical dosage form according to the claim 7 or 8 **characterized in that** in a dissolution test after 45 minutes at least 75 % by weight of the first and at least 75 % by weight of the second active pharmaceutical ingredient are dissolved.

**10.** The pharmaceutical dosage form according to the claim 7, 8 or 9 **characterized in that** in a disintegration test the pharmaceutical dosage form is disintegrated within 30 minutes.

**11.** A process for the preparation of a pharmaceutical dosage form according to one or more of the claim 7 to 10 comprising one or more granulation processes wherein the two active pharmaceutical ingredients together with one or more excipients are granulated.

**12.** The pharmaceutical composition according to one of the claims 1 to 6 for use in a method for

- preventing, slowing the progression of, delaying or treating a metabolic disorder selected from the group consisting of type 1 diabetes mellitus, type 2 diabetes mellitus, impaired glucose tolerance, impaired fasting blood glucose, hyperglycemia, postprandial hyperglycemia, overweight, obesity and metabolic syndrome; or
- improving glycemic control and/or for reducing of fasting plasma glucose, of postprandial plasma glucose and/or of glycosylated hemoglobin HbA1c; or
- preventing, slowing, delaying or reversing progression from impaired glucose tolerance, insulin resistance and/or from metabolic syndrome to type 2 diabetes mellitus; or
- preventing, slowing the progression of, delaying or treating of a condition or disorder selected from the group consisting of complications of diabetes mellitus such as cataracts and micro- and macrovascular diseases, such as nephropathy, retinopathy, neuropathy, tissue ischaemia, arteriosclerosis, myocardial infarction, stroke and peripheral arterial occlusive disease; or
- reducing body weight or preventing an increase in body weight or facilitating a reduction in body weight; or
- preventing, slowing, delaying or treating the degeneration of pancreatic beta cells and/or the decline of the functionality of pancreatic beta cells and/or for improving and/or restoring the functionality of pancreatic beta cells and/or restoring the functionality of pancreatic insulin secretion; or
- for preventing, slowing, delaying or treating diseases or conditions attributed to an abnormal accumulation of liver fat; or
- maintaining and/or improving the insulin sensitivity and/or for treating or preventing hyperinsulinemia and/or insulin resistance;

in a patient in need thereof.

**13.** The pharmaceutical composition according to claim 12 wherein the patient is an individual who shows one, two or more of the following conditions:

(a) a fasting blood glucose or serum glucose concentration greater than 100 mg/dL, in particular greater than 125 mg/dL;
(b) a postprandial plasma glucose equal to or greater than 140 mg/dL;
(c) an HbA1 c value equal to or greater than 6.5 %, in particular equal to or greater than 7.0 %.

**14.** The pharmaceutical composition according to claim 12 wherein the patient is an individual wherein one, two, three or more of the following conditions are present:

(a) obesity, visceral obesity and/or abdominal obesity,
(b) triglyceride blood level ≥ 150 mg/dL,
(c) HDL-cholesterol blood level < 40 mg/dL in female patients and < 50 mg/dL in male patients,
(d) a systolic blood pressure ≥ 130 mm Hg and a diastolic blood pressure ≥ 85 mm Hg,
(e) a fasting blood glucose level ≥ 100 mg/dL.

**15.** The pharmaceutical composition according to claim 12 wherein the patient has insufficient glycemic control despite diet and exercise or despite monotherapy with an antidiabetic agent.

**Figure 1:** X-ray powder diffraction pattern of the crystalline form

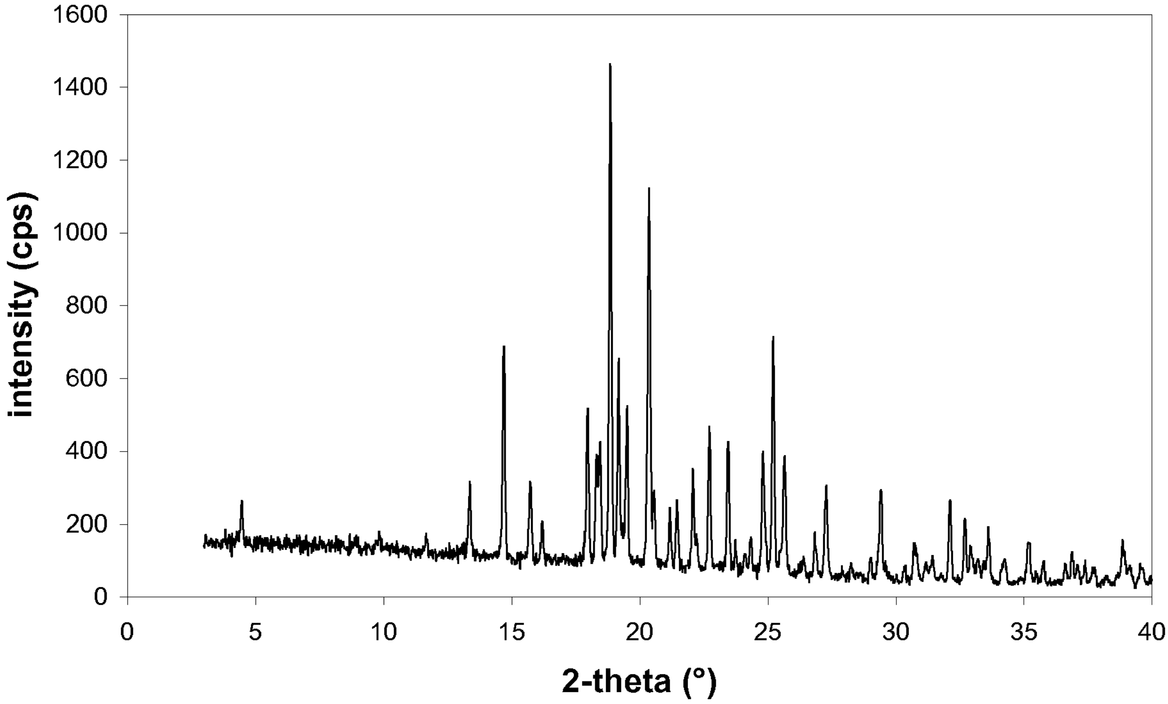


**Figure 2:** DSC and TG diagram of the crystalline form

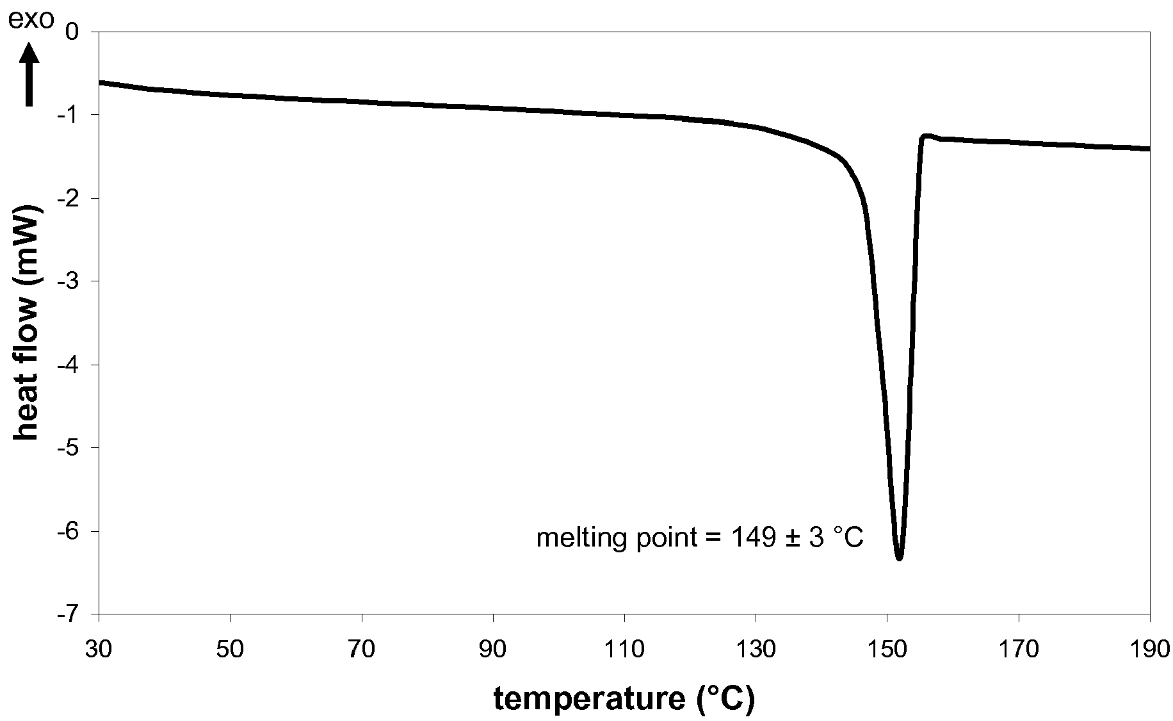

Figure 3

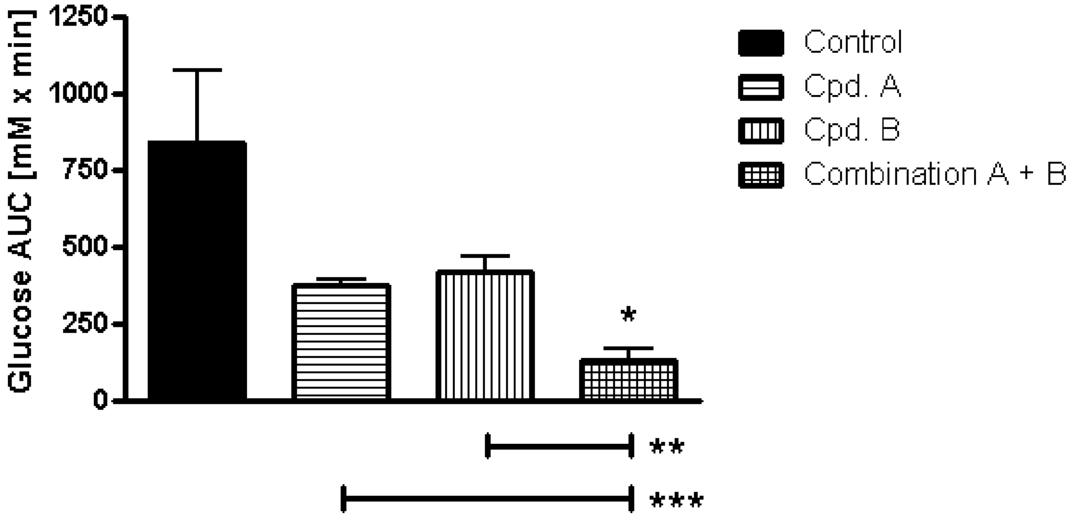


Figure 4

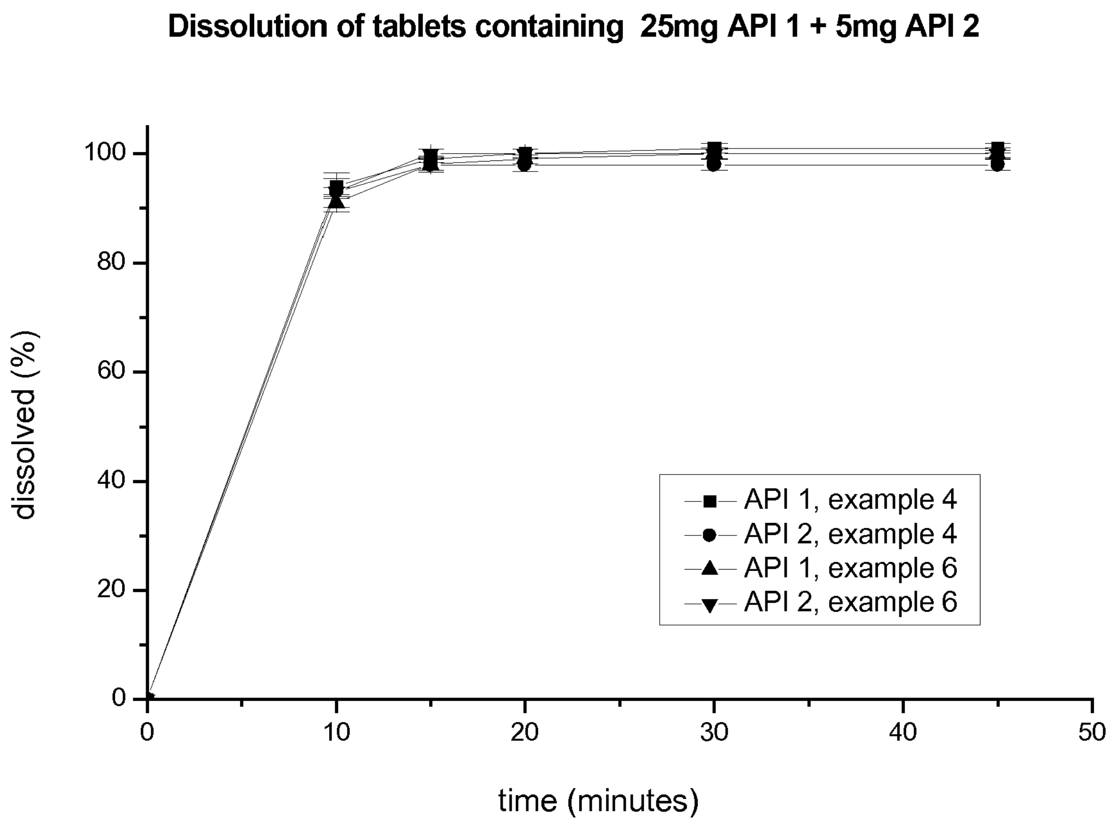

Figure 5:

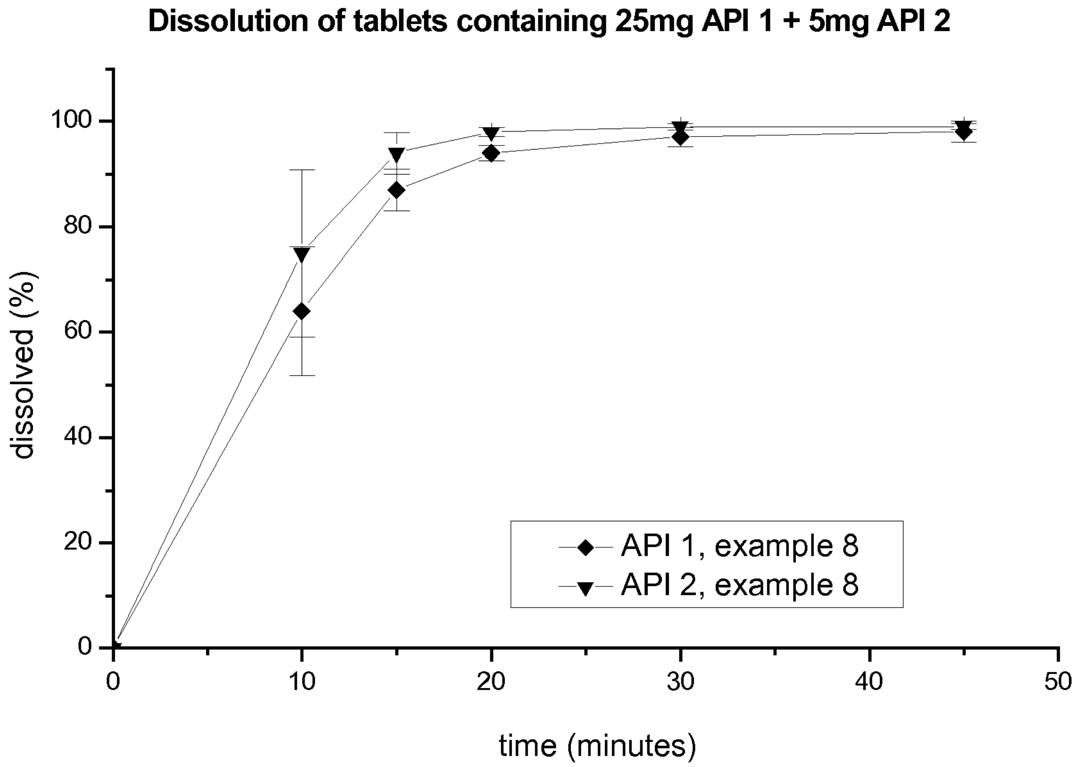
Dissolution of tablets containing 25mg API 1 + 5mg API 2
dissolved (%)
0
20
40
60
80
100
0
10
20
30
40
50
time (minutes)
API 1, example 8
API 2, example 8

Europäisches Patentamt
European Patent Office
Office européen des brevets

# EUROPEAN SEARCH REPORT

Application Number
EP 16 18 7550

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2009/022007 A1 (BOEHRINGER INGELHEIM INT [DE]; DUGI KLAUS [DE]; MARK MICHAEL [DE]; THO) 19 February 2009 (2009-02-19)<br>* page 23, line 20 - page 24 *<br>* page 13, line 5 - page 17, line 17 *<br>* claims *<br>* Combination 97 and 121 in Table 1 *<br>* page 68 - page 70 *<br>* page 41, line 23 - line 28 *<br>----- | 1,4-8, 11-15 | INV.<br>A61K31/7048<br>A61K31/522<br>A61P3/06<br>A61P3/08<br>A61P3/10<br>A61K9/20 |
| X,P | WO 2009/022010 A1 (BOEHRINGER INGELHEIM INT [DE]; DUGI KLAUS [DE]; MARK MICHAEL [DE]; HIM) 19 February 2009 (2009-02-19)<br>* page 21, lines 20-21 *<br>* page 8, line 4 *<br>* claims 1-22 *<br>* Combination 37 of Table 1 *<br>----- | 1,4-7, 11-15 | |
| Y | WANG Y ET AL: "BI-1356. Dipeptidyl-peptidase IV inhibitor, antidiabetic agent",<br>DRUGS OF THE FUTURE, PROUS SCIENCE, ES LNKD- DOI:10.1358/DOF.2008.033.06.1215244,<br>vol. 33, no. 6, 1 June 2008 (2008-06-01), pages 473-477, XP009110227,<br>ISSN: 0377-8282<br>* the whole document *<br>----- | 1-15 | TECHNICAL FIELDS SEARCHED (IPC)<br>A61K<br>A61P |
| Y | EP 1 852 108 A1 (BOEHRINGER INGELHEIM PHARMA [DE]) 7 November 2007 (2007-11-07)<br>* page 3, lines 44-55; claims *<br>----- | 1-15 | |
| Y | WO 2004/018468 A2 (BOEHRINGER INGELHEIM PHARMA [DE]; HIMMELSBACH FRANK [DE]; LANGKOPF ELK) 4 March 2004 (2004-03-04)<br>* example 2(80) *<br>-----<br>-/-- | 1-15 | |

The present search report has been drawn up for all claims

3

| Place of search | Date of completion of the search | Examiner |
|---|---|---|
| Munich | 24 January 2017 | Pacreu Largo, Marta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 18 7550

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2008/055870 A1 (BOEHRINGER INGELHEIM INT [DE]; BOEHRINGER INGELHEIM PHARMA [DE]; HIMME) 15 May 2008 (2008-05-15) * page 32, line 33 - page 33, paragraph 12 * * page 34, lines 10-35 * * claims * ----- | 1-15 | |
| Y,D | WO 2008/049923 A1 (BOEHRINGER INGELHEIM INT [DE]; BOEHRINGER INGELHEIM PHARMA [DE]; ECKHA) 2 May 2008 (2008-05-02) * claims * ----- | 1-15 | |
| Y,D | WO 2006/117359 A1 (BOEHRINGER INGELHEIM INT [DE]; BOEHRINGER INGELHEIM PHARMA [DE]; ECKHA) 9 November 2006 (2006-11-09) * claims * ----- | 1-15 | |

TECHNICAL FIELDS SEARCHED (IPC)

The present search report has been drawn up for all claims

3

| Place of search | Date of completion of the search | Examiner |
|---|---|---|
| Munich | 24 January 2017 | Pacreu Largo, Marta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 18 7550

This annex lists the patent family members relating to the patent documents cited in the above-mentioned European search report. The members are as contained in the European Patent Office EDP file on The European Patent Office is in no way liable for these particulars which are merely given for the purpose of information.

24-01-2017

| Patent document cited in search report | Publication date | Patent family member(s) | Publication date |
|---|---|---|---|
| WO 2009022007 A1 | 19-02-2009 | AR 067970 A1 | 28-10-2009 |
| | | AR 087657 A2 | 09-04-2014 |
| | | AU 2008288407 A1 | 19-02-2009 |
| | | CA 2696558 A1 | 19-02-2009 |
| | | CN 101784270 A | 21-07-2010 |
| | | CN 104288166 A | 21-01-2015 |
| | | CN 104353077 A | 18-02-2015 |
| | | CO 6251239 A2 | 21-02-2011 |
| | | DK 2187879 T3 | 09-01-2017 |
| | | EA 201000321 A1 | 30-08-2010 |
| | | EC SP109977 A | 31-03-2010 |
| | | EP 2187879 A1 | 26-05-2010 |
| | | EP 2698152 A1 | 19-02-2014 |
| | | EP 3106156 A1 | 21-12-2016 |
| | | HK 1201721 A1 | 11-09-2015 |
| | | HK 1203351 A1 | 30-10-2015 |
| | | IL 202886 A | 28-11-2013 |
| | | JP 5595914 B2 | 24-09-2014 |
| | | JP 2010535850 A | 25-11-2010 |
| | | KR 20100049595 A | 12-05-2010 |
| | | MA 31612 B1 | 02-08-2010 |
| | | MY 152037 A | 15-08-2014 |
| | | NZ 583242 A | 26-10-2012 |
| | | PE 09382009 A1 | 08-08-2009 |
| | | TN 2010000073 A1 | 26-09-2011 |
| | | TW 200914030 A | 01-04-2009 |
| | | TW 201436798 A | 01-10-2014 |
| | | UA 100384 C2 | 25-12-2012 |
| | | US 2011195917 A1 | 11-08-2011 |
| | | UY 31296 A1 | 31-03-2009 |
| | | WO 2009022007 A1 | 19-02-2009 |
| | | ZA 200909105 B | 29-09-2010 |
| WO 2009022010 A1 | 19-02-2009 | AR 067969 A1 | 28-10-2009 |
| | | AU 2008288410 A1 | 19-02-2009 |
| | | BR PI0815170 A2 | 31-03-2015 |
| | | CA 2696271 A1 | 19-02-2009 |
| | | CN 101784286 A | 21-07-2010 |
| | | EP 2187966 A1 | 26-05-2010 |
| | | JP 2010536734 A | 02-12-2010 |
| | | KR 20100055422 A | 26-05-2010 |
| | | NZ 583240 A | 26-10-2012 |
| | | PE 06032009 A1 | 11-06-2009 |
| | | RU 2010109449 A | 20-10-2011 |
| | | TW 200914031 A | 01-04-2009 |
| | | US 2011098240 A1 | 28-04-2011 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 16 18 7550

This annex lists the patent family members relating to the patent documents cited in the above-mentioned European search report. The members are as contained in the European Patent Office EDP file on The European Patent Office is in no way liable for these particulars which are merely given for the purpose of information.

24-01-2017

| Patent document cited in search report | Publication date | Patent family member(s) | Publication date |
|---|---|---|---|
| | | US 2013096076 A1 | 18-04-2013 |
| | | UY 31295 A1 | 31-03-2009 |
| | | WO 2009022010 A1 | 19-02-2009 |
| | | ZA 200908992 B | 25-08-2010 |
| EP 1852108 A1 | 07-11-2007 | AR 060755 A1 | 10-07-2008 |
| | | AR 079930 A2 | 29-02-2012 |
| | | AT 480228 T | 15-09-2010 |
| | | AU 2007247193 A1 | 15-11-2007 |
| | | BR PI0711179 A2 | 03-05-2011 |
| | | BR PI0722388 A2 | 19-05-2015 |
| | | CA 2649922 A1 | 15-11-2007 |
| | | CL 2012002521 A1 | 21-12-2012 |
| | | CL 2012002522 A1 | 21-12-2012 |
| | | CN 101437493 A | 20-05-2009 |
| | | CN 102526737 A | 04-07-2012 |
| | | CY 1111354 T1 | 05-08-2015 |
| | | DK 2023902 T3 | 15-11-2010 |
| | | DK 2277509 T3 | 13-04-2015 |
| | | DK 2283819 T3 | 05-01-2015 |
| | | EA 200802184 A1 | 30-06-2009 |
| | | EA 201100958 A1 | 30-04-2012 |
| | | EC SP088800 A | 27-11-2008 |
| | | EP 1852108 A1 | 07-11-2007 |
| | | EP 2023902 A1 | 18-02-2009 |
| | | EP 2277509 A1 | 26-01-2011 |
| | | EP 2283819 A1 | 16-02-2011 |
| | | EP 2910241 A1 | 26-08-2015 |
| | | ES 2348576 T3 | 09-12-2010 |
| | | ES 2527409 T3 | 23-01-2015 |
| | | ES 2538818 T3 | 24-06-2015 |
| | | HK 1130442 A1 | 11-07-2014 |
| | | HK 1172549 A1 | 18-12-2015 |
| | | HR P20100507 T1 | 31-10-2010 |
| | | HR P20150003 T1 | 13-03-2015 |
| | | IL 195030 A | 30-09-2013 |
| | | JP 5478244 B2 | 23-04-2014 |
| | | JP 2009535376 A | 01-10-2009 |
| | | JP 2012072187 A | 12-04-2012 |
| | | JP 2013227338 A | 07-11-2013 |
| | | JP 2016104811 A | 09-06-2016 |
| | | KR 20090009226 A | 22-01-2009 |
| | | KR 20140063896 A | 27-05-2014 |
| | | KR 20150100957 A | 02-09-2015 |
| | | KR 20160128446 A | 07-11-2016 |
| | | MY 146969 A | 15-10-2012 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 16 18 7550

This annex lists the patent family members relating to the patent documents cited in the above-mentioned European search report. The members are as contained in the European Patent Office EDP file on
The European Patent Office is in no way liable for these particulars which are merely given for the purpose of information.

24-01-2017

| Patent document cited in search report | Publication date | Patent family member(s) | Publication date |
|---|---|---|---|
| | | MY 148496 A | 30-04-2013 |
| | | NZ 572862 A | 25-11-2011 |
| | | NZ 595983 A | 30-08-2013 |
| | | NZ 613426 A | 27-02-2015 |
| | | PE 06662011 A1 | 23-09-2011 |
| | | PE 06982008 A1 | 04-08-2008 |
| | | PT 2023902 E | 12-10-2010 |
| | | PT 2283819 E | 03-11-2014 |
| | | RS 51466 B | 30-04-2011 |
| | | SG 171649 A1 | 29-06-2011 |
| | | SI 2023902 T1 | 31-01-2011 |
| | | SI 2283819 T1 | 30-01-2015 |
| | | TW 200812648 A | 16-03-2008 |
| | | TW 201417844 A | 16-05-2014 |
| | | UA 94942 C2 | 25-06-2011 |
| | | US 2008107731 A1 | 08-05-2008 |
| | | US 2012003313 A1 | 05-01-2012 |
| | | US 2012219622 A1 | 30-08-2012 |
| | | US 2013122089 A1 | 16-05-2013 |
| | | US 2016022687 A1 | 28-01-2016 |
| | | UY 30319 A1 | 02-01-2008 |
| | | WO 2007128724 A1 | 15-11-2007 |
| | | ZA 200808361 B | 27-10-2010 |
| WO 2004018468 A2 | 04-03-2004 | AR 041025 A1 | 27-04-2005 |
| | | AR 077875 A2 | 28-09-2011 |
| | | AR 087287 A2 | 12-03-2014 |
| | | AT 453644 T | 15-01-2010 |
| | | AU 2003253418 A1 | 11-03-2004 |
| | | AU 2010201384 A1 | 29-04-2010 |
| | | BR 0313648 A | 08-05-2007 |
| | | CA 2496249 A1 | 04-03-2004 |
| | | CN 1675212 A | 28-09-2005 |
| | | CN 102964347 A | 13-03-2013 |
| | | CN 105001222 A | 28-10-2015 |
| | | CY 1109928 T1 | 10-09-2014 |
| | | DE 122011100059 I1 | 05-04-2012 |
| | | DK 1532149 T3 | 10-05-2010 |
| | | EA 200702022 A1 | 30-06-2008 |
| | | EA 201001167 A1 | 28-02-2011 |
| | | EC SP055617 A | 30-05-2005 |
| | | EC SP12005617 A | 30-04-2012 |
| | | EG 25011 A | 25-05-2011 |
| | | EP 1532149 A2 | 25-05-2005 |
| | | EP 2058311 A2 | 13-05-2009 |
| | | EP 2060573 A2 | 20-05-2009 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 16 18 7550

This annex lists the patent family members relating to the patent documents cited in the above-mentioned European search report. The members are as contained in the European Patent Office EDP file on The European Patent Office is in no way liable for these particulars which are merely given for the purpose of information.

24-01-2017

| Patent document cited in search report | Publication date | Patent family member(s) | Publication date |
|---|---|---|---|
| | | EP 2070539 A1 | 17-06-2009 |
| | | EP 2308878 A2 | 13-04-2011 |
| | | ES 2339112 T3 | 17-05-2010 |
| | | HK 1081552 A1 | 20-11-2009 |
| | | HK 1134076 A1 | 25-04-2014 |
| | | HK 1134077 A1 | 12-09-2014 |
| | | HK 1183028 A1 | 25-11-2016 |
| | | HK 1215439 A1 | 26-08-2016 |
| | | HR P20050157 A2 | 31-05-2006 |
| | | HR P20090665 A2 | 31-07-2010 |
| | | IL 166964 A | 28-02-2011 |
| | | JP 4233524 B2 | 04-03-2009 |
| | | JP 4971251 B2 | 11-07-2012 |
| | | JP 5351486 B2 | 27-11-2013 |
| | | JP 5623459 B2 | 12-11-2014 |
| | | JP 2006503013 A | 26-01-2006 |
| | | JP 2009001565 A | 08-01-2009 |
| | | JP 2009079061 A | 16-04-2009 |
| | | JP 2012162555 A | 30-08-2012 |
| | | JP 2014177485 A | 25-09-2014 |
| | | JP 2016199572 A | 01-12-2016 |
| | | KR 20050058375 A | 16-06-2005 |
| | | KR 20090047560 A | 12-05-2009 |
| | | LU 91889 I2 | 21-12-2011 |
| | | LU 92128 I2 | 11-03-2013 |
| | | ME P59708 A | 10-05-2011 |
| | | MX PA05001684 A | 19-04-2005 |
| | | MY 137619 A | 27-02-2009 |
| | | NO 333970 B1 | 04-11-2013 |
| | | NO 336641 B1 | 12-10-2015 |
| | | PE 08972004 A1 | 31-01-2005 |
| | | PL 216134 B1 | 31-03-2014 |
| | | PT 1532149 E | 17-02-2010 |
| | | RS 20050137 A | 15-11-2007 |
| | | RS 20120139 A2 | 31-10-2012 |
| | | SI 1532149 T1 | 31-05-2010 |
| | | TW I319320 B | 11-01-2010 |
| | | TW 200944209 A | 01-11-2009 |
| | | TW 201304782 A | 01-02-2013 |
| | | UA 84275 C2 | 10-10-2008 |
| | | UY 27946 A1 | 31-03-2004 |
| | | WO 2004018468 A2 | 04-03-2004 |
| WO 2008055870 A1 | 15-05-2008 | AR 063569 A1 | 04-02-2009 |
| | | CA 2668623 A1 | 15-05-2008 |
| | | CL 2007003187 A1 | 07-03-2008 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 16 18 7550

This annex lists the patent family members relating to the patent documents cited in the above-mentioned European search report. The members are as contained in the European Patent Office EDP file on
The European Patent Office is in no way liable for these particulars which are merely given for the purpose of information.

24-01-2017

| Patent document cited in search report | Publication date | Patent family member(s) | Publication date |
|---|---|---|---|
| | | EP 2079753 A1 | 22-07-2009 |
| | | JP 2010508371 A | 18-03-2010 |
| | | TW 200829258 A | 16-07-2008 |
| | | US 2010069310 A1 | 18-03-2010 |
| | | WO 2008055870 A1 | 15-05-2008 |
| WO 2008049923 A1 | 02-05-2008 | CA 2667550 A1 | 02-05-2008 |
| | | EP 2086991 A1 | 12-08-2009 |
| | | JP 2010507629 A | 11-03-2010 |
| | | US 2010317847 A1 | 16-12-2010 |
| | | WO 2008049923 A1 | 02-05-2008 |
| WO 2006117359 A1 | 09-11-2006 | AR 053720 A1 | 16-05-2007 |
| | | AR 087208 A2 | 26-02-2014 |
| | | AT 452883 T | 15-01-2010 |
| | | AU 2006243859 A1 | 09-11-2006 |
| | | BR PI0610994 A2 | 10-08-2010 |
| | | CA 2606650 A1 | 09-11-2006 |
| | | CY 1109870 T1 | 10-09-2014 |
| | | DK 1888552 T3 | 12-04-2010 |
| | | EA 200702346 A1 | 28-04-2008 |
| | | EP 1888552 A1 | 20-02-2008 |
| | | EP 2166007 A1 | 24-03-2010 |
| | | ES 2337498 T3 | 26-04-2010 |
| | | HK 1115133 A1 | 23-12-2011 |
| | | HR P20100033 T1 | 31-03-2010 |
| | | IL 187087 A | 30-06-2011 |
| | | JP 4226070 B2 | 18-02-2009 |
| | | JP 2008540373 A | 20-11-2008 |
| | | JP 2009046513 A | 05-03-2009 |
| | | KR 20080015424 A | 19-02-2008 |
| | | MY 142108 A | 15-09-2010 |
| | | NO 339073 B1 | 07-11-2016 |
| | | NZ 563563 A | 26-11-2010 |
| | | PE 10632009 A1 | 19-08-2009 |
| | | PE 13742006 A1 | 09-01-2007 |
| | | PT 1888552 E | 02-03-2010 |
| | | SI 1888552 T1 | 30-04-2010 |
| | | TW I344465 B | 01-07-2011 |
| | | US 2007249544 A1 | 25-10-2007 |
| | | US 2010099641 A1 | 22-04-2010 |
| | | UY 29505 A1 | 30-11-2006 |
| | | WO 2006117359 A1 | 09-11-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007128724 A
- WO 0127128 A
- WO 03099836 A
- WO 2005092877 A
- WO 2006034489 A
- WO 2006064033 A
- WO 2006117359 A
- WO 2006117360 A
- WO 2007025943 A
- WO 2007028814 A
- WO 2007031548 A
- WO 2007093610 A
- WO 2007128749 A
- WO 2008049923 A
- WO 2008055870 A
- WO 2008055940 A
- WO 2007128721 A
- WO 2004018468 A
- WO 2006048427 A
- WO 2006120208 A


### Non-patent literature cited in the description


- **FORD ES et al.** *JAMA,* 2002, vol. 287, 356-9
- **KATSUKI A et al.** *Diabetes Care,* 2001, vol. 24, 362-5
- **MATTHEWS et al.** *Diabetologia,* 1985, vol. 28, 412-19
- **FORST et al.** *Diabetes,* 2003, vol. 52 (1), A459
- **GALVIN P et al.** *Diabet Med,* 1992, vol. 9, 921-8
- **STUMVOLL et al.** *Eur J Clin Invest,* 2001, vol. 31, 380-81
- **J. B. MEIGS et al.** *Diabetes,* 2003, vol. 52, 1475-1484
- *Diabetes Care,* 2002, vol. 25, 742-749
- **LAAKSONEN DE et al.** *Am J Epidemiol,* 2002, vol. 156, 1070-7
- *JAMA: Journal of the American Medical Association,* 2001, vol. 285, 2486-2497
- **LAAKSONEN DE et al.** *Am J Epidemiol.,* 2002, vol. 156, 1070-7
- TH-Books Verlagsgesellschaft mbH, Frankfurt/Main. Labor und Diagnose. 2000
- United States Pharmacopeial Convention. *USP 31-NF 26 through Second Supplement*
- European Directorate for the Quality of Medicines and Health Care. European Pharmacopoeia 6.3. 2000